(19) Europäisches Patentamt
European Patent Office
Office européen des brevets

(11) Numéro de publication : **0 432 000 B1**

(12) # FASCICULE DE BREVET EUROPEEN

(45) Date de publication du fascicule du brevet : **11.08.93 Bulletin 93/32**

(51) Int. Cl.$^5$ : **A61K 7/09,** C07C 323/25, C07C 327/30

(21) Numéro de dépôt : **90403267.9**

(22) Date de dépôt : **20.11.90**

(54) **Composition réductrice, pour déformation permanente des cheveux, contenant en tant qu'agent réducteur un amino mercaptoalkylamide ou l'un de ses sels, et son utilisation dans un procédé de déformation permanente des cheveux.**

(30) Priorité : **20.11.89 FR 8915182**

(43) Date de publication de la demande : **12.06.91 Bulletin 91/24**

(45) Mention de la délivrance du brevet : **11.08.93 Bulletin 93/32**

(84) Etats contractants désignés : **AT BE CH DE DK ES GB GR IT LI NL SE**

(56) Documents cités :
**EP-A- 0 354 835**
**GB-A- 891 600**
**US-A- 3 768 490**
**PATENT ABSTRACTS OF JAPAN, vol. 6, no. 14 (C-131)[1092], 27 octobre 1982; & JP-A-57 118 556 (DAIICHI SEIYAKU K.K.) 23-07-1982 Résumé**
**PATENT ABSTRACT OF JAPAN, vol. 4, no. 34 (C-3)[516], 22 mars 1980; & JP-A-55 7222 (DAIICHI SEIYAKU K.K.) 19-01-1980**

(56) Documents cités :
**CHEMICAL ABSTRACTS, 83, no. 21, 24 novembre 1975, page 511, résumé no. 178345t, Columbus, Ohio, US; && JP-A-75 62 931 (DAIICHI SEIYAKU CO., LTD) 29-05-1975**
**CHEMICAL ABSTRACTS, vol. 103, no. 12, 23 septembre 1985, page 512, résumé no. 95577a, Columbus, Ohio, US; E.R.J. WILS et al.: "Mass spectra of the S-(2-aminoethyl) ester of 3-phenylthioalanine-DL and its acetylated derivatives", & ORG. MASS SPECTROM. 1985, 20(5), 381-2**

(73) Titulaire : **L'OREAL**
**14, Rue Royale**
**F-75008 Paris (FR)**

(72) Inventeur : **Junino, Alex**
**162, avenue Vauban**
**F-93190 Livry Gargan (FR)**
Inventeur : **Malle, Gérard**
**18, Grande rue**
**F-77580 Villiers S/Morin (FR)**

(74) Mandataire : **Nony, Michel et al**
**Cabinet NONY & CIE 29, rue Cambacérès**
**F-75008 Paris (FR)**

**EP 0 432 000 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention a pour objet une composition réductrice, pour le premier temps d'une opération de déformation permanente des cheveux, contenant en tant qu'agent réducteur un amino mercaptoalkylamide ou l'un de ses sels cosmétiquement acceptables, et son utilisation dans un procédé de déformation permanente des cheveux.

La technique pour réaliser la déformation permanente des cheveux consiste, dans un premier temps, à réaliser l'ouverture des liaisons disulfures de la kératine (cystine) à l'aide d'une composition contenant un agent réducteur (étape de réduction), puis, après avoir de préférence rincé la chevelure, à reconstituer dans une second temps lesdites liaisons disulfures en appliquant, sur les cheveux sous tension, une composition oxydante, (étape d'oxydation, dite aussi de fixation) de façon à donner aux cheveux la forme recherchée. Cette technique permet indifféremment de réaliser soit l'ondulation des cheveux, soit leur défrisage ou leur décrêpage.

Les compositions pour réaliser le premier temps d'une opération de permanente se présentent généralement sous forme de lotions, de crèmes, de gels ou de poudres à diluer dans un support liquide et contiennent, en tant qu'agent réducteur, de préférence un mercaptan.

Parmi ces derniers, ceux couramment utilisés sont l'acide thioglycolique et l'acide thiolactique ou un mélange de ces acides ainsi que leurs esters par exemple le monothioglycolate de glycérol ou de glycol.

Ces agents réducteurs sont particulièrement efficaces pour réduire les liaisons disulfures de la kératine notamment l'acide thioglycolique qui peut être considéré comme le produit de référence en permanente, et qui conduit à un taux de réduction d'environ 50 %.

Ces agents réducteurs présentent cependant un inconvénient majeur dans la mesure où ils dégagent de mauvaises odeurs.

En vue d'y remédier, il est généralement fait usage d'un parfum permettant de masquer les odeurs.

Après d'importantes recherches, on a maintenant constaté, de façon tout à fait inattendue et surprenante, qu'en utilisant une nouvelle famille d'amino mercaptoalkylamides ou leurs sels cosmétiquement acceptables, il était possible de remédier aux inconvénients des agents réducteurs de l'état de la technique.

Les agents réducteurs des compositions, selon l'invention, présentent non seulement l'avantage d'être pratiquement dépourvus d'odeur mais permettent également d'obtenir un rendement, une nervosité et une beauté de frisure supérieurs à ceux obtenus selon l'état de la technique à l'aide par exemple de l'acide thioglycolique.

La présente invention a donc pour objet à titre de produit industriel nouveau, une composition cosmétique pour le premier temps d'une opération de déformation permanente des cheveux, contenant, dans un véhicule cosmétique approprié, en tant qu'agent réducteur, au moins un amino mercaptoalkylamide répondant à la formule générale (I) suivante :

$$HS{-}A{-}NH{-}CO{-}\underset{\displaystyle R_1}{\underset{|}{C}H}{-}(CH_2)_m{-}NH_2 \qquad (I)$$

dans laquelle :

A représente le radical divalent $-(CH_2)_n-$, n étant un nombre entier compris entre 2 et 5, ou le radical divalent $-(CH_2)_2-O-(CH_2)_2-$ m est 0 ou un nombre entier compris entre 1 et 4,

(i) lorsque m est O, $R_1$ représente un atome d'hydrogène, un radical méthyle, un radical éthyle, un radical isopropyle, un radical isobutyle, un radical méthyl-2-butyle, un radical benzyle, un radical amino-4 butyle, un radical guanidino-3 propyle, un radical méthylthio-2 éthyle, un radical carboxyméthyle ou un radical carboxy-2 éthyle,

(ii) lorsque m est un nombre entier de 1 à 4, $R_1$ représente un atome d'hydrogène ou un radical alkyle inférieur, linéaire ou ramifié, ayant de 1 à 5 atomes de carbone,

et les sels desdits composés de formule (I).

Parmi les sels cosmétiquement acceptables des composés de formule (I) ceux particulièrement préférés sont les chlorhydrates, bromhydrates, citrates et oxalates.

Par radical alkyle inférieur ayant de 1 à 5 atomes de carbone, on doit entendre un radical méthyle, éthyle, propyle, isopropyle, butyle, isobutyle, méthyle-2 butyle ou pentyle.

Parmi les composés préférés répondant à la formule générale (I) on peut notamment citer ;

- l'amino-3 N-(mercapto-2 éthyl) propionamide ou aléthéine,
- l'amino-2 N-(mercapto-2 éthyl) acétamide,
- l'amino-2 N-(mercapto-3 propyl) acétamide,

- l'amino-2 N-(mercapto-5 pentyl) acétamide,
- l'amino-2 N-[(mercapto-2 éthoxy)-2'éthyl]acétamide,
- l'amino-2 N-(mercapto-2 éthyl) propionamide,
- l'amino-2 N-(mercapto-3 propyl) propionamide,
- l'amino-2 N-(mercapto-5 pentyl) propionamide,
- l'amino-2 N-[(mercapto-2 éthoxy-2') éthyl]propionamide,
- l'amino-3 N-(mercapto-3 propyl) propionamide,
- l'amino-3 N-(mercapto-5 pentyl) propionamide,
- l'amino-3 N- (mercapto-2 éthoxy)-2' éthyl propionamide,
- l'amino-2 N-(mercapto-2 éthyl) méthyl-3 butanamide,
- l'amino-2 N-(mercapto-2 éthyl) méthyl-4 pentanamide,
- l'αamino N-(mercapto-2 éthyl) hydrocinnamide,
- l'acide amino-4[(mercapto-2 éthyl) amino]-5 oxo-5 pentanoïque,
- l'amino-4 N-(mercapto-2 éthyl) butanamide,
- l'amino-4 N-(mercapto-3 propyl) butanamide,
- l'amino-6 N-(mercapto-5 pentyl) butanamide,
- l'amino-4[N-(mercapto-2 éthoxy)-2' éthyl]butanamide, et
- l'amino-6 N-(mercapto-2 éthyl) hexanamide,

ainsi que leurs chlorhydrates.

Les composés de formule générale (I), dont certains sont connus, sont préparés suivant des méthodes connues en soi que l'on mentionnera ci-après et qui dépendent des différents substituants, des matières premières utilisées et de leur facilité de mise en oeuvre.

Les composés selon l'invention peuvent notamment être préparés par l'intermédiaire des thioesters de formule générale (II) :

$$H_2N-A-S-CO-\underset{\displaystyle R_1}{\underset{|}{C}}H-(CH_2)_m-NH_2\ ,\ 2HX \qquad (II)$$

dans laquelle :

A, $R_1$ et m ont les mêmes significations que ci-dessus pour la formule générale (I) et,

X est Cl ou Br.

Ces thioesters sont obtenus, soit par condensation d'un aminothiol sur un halogénure d'acide d'amino-acide tel que décrit par exemple par Th. WIELAND et al, Justus Liebigs Annalen der Chemie, 576, 20, (1952), soit par hydrolyse d'une thiazoline substituée, celle-ci étant obtenue par réaction d'un aminonitrile sur un aminothiol tel que la cystéamine, comme décrit dans les demandes de brevets japonais 66/22389, 75/62931 et 75/62932 de la Société DAICHI SEYAKU Company.

Les thioesters de formule générale (II) sont réarrangés en amino mercaptoalkylamides de formule générale (I) par traitement avec une base.

Cette réaction est conduite en milieu hydroalcoolique à température ambiante en présence d'au moins 1 équivalent d'une base telle que l'ammoniaque, les hydroxydes de métaux alcalins ou encore une amine tertiaire telle que la triéthylamine ou la triéthanolamine selon la réaction suivante :

$$H_2N-A-S-CO-\underset{\displaystyle R_1}{\underset{|}{C}}H-(CH_2)_m-NH_2,\ 2HX \quad \xrightarrow{\text{base}}$$

(II)

$$HS-A-NH-CO-\underset{\displaystyle R_1}{\underset{|}{C}}H-(CH_2)_m-NH_2,\ HX$$

(I)

Les composés selon l'invention peuvent également être préparés de façon plus classique par condensation d'un aminoacide N-protégé avec un S-acylaminothiol en présence d'un agent d'activation de synthèse peptidique tel que par exemple le dicyclohexylcarbodiimide.

Les fonctions thiol et amine sont ensuite déprotégées par hydrolyse acide ou basique, tel que décrit par exemple par M. FATOME et al. Eur. J. Med. Chem., 23, (1988), 257-266 ou encore par J. OIRY et al., J. Med. Chem., 29 (11), 2217-25, (1986).

Dans les compositions selon l'invention l'agent réducteur de formule générale (I) est généralement présent à une concentration comprise entre 2 et 30 % et de préférence entre 5 et 25 % en poids par rapport au poids total de la composition réductrice.

Le pH de la composition est de préférence compris entre 4,5 et 11 et plus particulièrement entre 6 et 10 et est obtenu à l'aide d'un agent alcalin tel que par exemple l'ammoniaque, le monoéthanolamine, la diéthanolamine, la triéthanolamine, un carbonate ou bicarbonate alcalin ou d'ammonium, un hydroxyde alcalin ou à l'aide d'un agent acidifiant tel que par exemple l'acide chlorhydrique, l'acide acétique, l'acide lactique, l'acide oxalique ou l'acide borique.

La composition réductrice peut également contenir d'autres agents réducteurs connus tels que par exemple l'acide thioglycolique, le monothioglycolate de glycérol ou de glycol, la cystéamine et ses dérivés acylés $C_1$-$C_4$ tels que la N-acétyl cystéamine ou la N-propionyl cystéamine, la cystéine, la N-acétylcystéine, les N-mercaptoalkylamides de sucres tels que la N-(mercapto-2-éthyl)gluconamide, l'acide β-mercaptopropionique et ses dérivés, l'acide thiolactique, la pantéthéine ou encore le thioglycérol, les sulfites, les bisulfites d'un métal alcalin ou alcalino-terreux et les thiols décrits dans les demandes de brevet EP 354.835 et EP 368.763.

La composition réductrice peut en outre contenir divers ingrédients tels que par exemple des polymères cationiques tels que ceux utilisés dans les compositions des brevets français n° 79.32078 et 80.26421 ou encore des polymères cationiques de type ionène tels que ceux utilisés dans les compositions du brevet français n° 82.17364, des agents adoucissants et notamment des ammonium quaternaires dérivés de la lanoline, des hydrolysats de protéines, des cires, des agents opacifiants, des parfums, des colorants, des tensio-actifs non ioniques ou cationiques, des alcools tels que l'éthanol, le propanol, l'isopropanol, le propanediol-1,2, le butanediol-1,2 ou le glycérol, des agents traitants ou encore des agents de pénétration tels que l'urée, la pyrrolidone ou la thiamorpholinone.

La composition réductrice selon l'invention peut être également du type exothermique, c'est-à-dire provoquant un certain échauffement lors de l'application sur les cheveux, ce qui apporte un agrément à la personne qui subit le premier temps de la permanente ou du défrisage.

Le véhicule des compositions selon l'invention est de préférence de l'eau ou une solution hydroalcoolique d'un alcool inférieur tel que l'éthanol, l'isopropanol ou le butanol.

Lorsque les compositions sont destinées à une opération de défrisage ou de décrêpage des cheveux, la composition réductrice est de préférence sous forme d'une crème de façon à maintenir les cheveux aussi raides que possible. On réalise ces crèmes, sous forme d'émulsions "lourdes", par exemple à base de stéarate de glycéryle, de stéarate de glycol, de cires auto-émulsionnables, d'alcools gras, etc...

On peut également utiliser des liquides ou des gels contenant des agents épaississants tels que des polymères ou des copolymères carboxyvinyliques qui "collent" les cheveux et les maintiennent dans la position lisse pendant le temps de pose.

Selon une forme de réalisation particulière de l'invention, les composés réducteurs de formule générale (I) peuvent être formés in situ au moment de l'emploi à partir de leurs précurseurs thioesters répondant à la formule générale (II) suivante :

$$H_2N-A-S-CO-\underset{\substack{|\\R_1}}{CH}-(CH_2)_m-NH_2 \quad , \ 2HX \qquad (II)$$

dans laquelle :

A, $R_1$ et m ont les mêmes significations que ci-dessus pour la formule générale (I), et

X est Cl ou Br.

On a en effet constaté qu'en présence d'une base telle que l'ammoniaque, la monoéthanolamine, la diéthanolamine, la triéthanolamine, la soude ou la potasse, ces précurseurs thioesters se transformaient quasi instantanément et quantitativement en thiols correspondants à la formule générale (I).

Selon cette forme de réalisation, le précurseur thioester à l'état solide, de préférence sous forme de poudre, est mélangé au moment de l'emploi à une solution aqueuse basique de pH compris entre 8 et 10, contenant

éventuellement divers ingrédients tels que ceux mentionnés ci-dessus.

Le pH de la composition est ensuite ajusté, si nécessaire, à l'aide d'un agent alcalin ou d'un agent acidifiant tels que ceux précédemment mentionnés.

Parmi ces thioesters on peut notamment citer les composés suivants :

- le dichlorhydrate de l'amino-3 thiopropionate d'amino-2 éthyle,
- le dichlorhydrate du thioglycinate d'amino-2 éthyle,
- le dichlorhydrate du thioalaninate d'amino-2 éthyle,
- le dichlorhydrate de l'amino-2 thiobutyrate d'amino-2 éthyle,
- le dichlorhydrate du thiovalinate d'amino-2 éthyle,
- le dichlorhydrate du thioleucinate d'amino-2 éthyle,
- le dichlorhydrate du phényl-3 thioalaninate d'amino-2 éthyle,
- le dichlorhydrate du thiométhioninate d'amino-2 éthyle,
- le dichlorhydrate du thioglycinate d'amino-3 propyle,
- le dichlorhydrate du thioglycinate d'amino-5 pentyle,
- le dichlorhydrate du thioglycinate d'(amino-2 éthoxy)-2-éthyle,
- le dichlorhydrate de l'amino-3 thiopropionate d'amino-3 propyle,
- le dichlorhydrate de l'amino-3 thiopropionate d'amino-5 pentyle,
- le dichlorhydrate de l'amino-3 thiopropionate d'(amino-2 éthoxy)-2' éthyle,
- le dichlorhydrate de l'amino-4 thiobutyrate d'amino-2 éthyle,
- le dichlorhydrate de l'amino-4 thiobutyrate d'amino-3 propyle,
- le dichlorhydrate de l'amino-4 thiobutyrate d'amino-5 pentyle,
- le dichlorhydrate de l'amino-4 thiobutyrate d'(amino-2 éthoxy)-2' éthyle,
- le dichlorhydrate de l'amino-6 thiocaproate d'amino-2 éthyle,
- le dichlorhydrate thioalaninate d'amino-3 propyle,
- le dichlorhydrate du thioalaninate d'amino-5 pentyle, et
- le dichlorhydrate du thioalaninate d'(amino-2 éthoxy)-2'éthyle.

Les compositions selon l'invention peuvent être également sous forme dite "auto-neutralisante" ou encore "auto-régulée" et dans ce cas, le composé de formule générale (I) est associé à au moins un disulfure soit connu pour son utilisation dans une composition réductrice pour permanente auto-neutralisante soit dérivant d'un composé de formule générale (I) ou de l'un de ses sels et correspondant à la formule générale (III) suivante :

$$\left[-S-A-NH-CO-\underset{\displaystyle R_1}{\underset{|}{C}}H-(CH_2)_m-NH_2\right]_2 \qquad (III)$$

dans laquelle :

A, $R_1$ et m ont les mêmes significations que ci-dessus pour la formule générale (I)

Ce disulfure peut également se présenter sous forme d'un sel cosmétiquement acceptable.

Parmi les disulfures connus, on peut notamment mentionner l'acide dithioglycolique, le dithioglycérol, la cystamine, la N,N'-diacétyl-cystamine, la cystine, la pantéthine, et les disulfures décrits dans les demandes de brevet européen EP 354.835 et EP 368.763.

Parmi les disulfures dérivant d'un composé de formule générale (I) et correspondant à la formule générale (III) on peut citer :

le N,N'-(dithiodiéthanediyl-2,1) bis [amino-3 propionamide],
le N,N'-(dithiodiéthanediyl-2,1) bis [amino-2 acétamide],
le N,N'-(dithiodiéthanediyl-2,1) bis [amino-2 propionamide],
le N,N'-[dithiobis (triméthylène)] bis [amino-2 propionamide],
le N,N'-(dithiodiéthanediyl-2,1)bis [α-amino benzène propionamide],
le N,N'-(dithiodiéthanediyl-2,1)bis [amino-2 méthyl-4 pentanamide],
le N,N'-(dithiodiéthanediyl-2,1)bis [amino-4 butanamide],
le N,N'-[dithiobis(triméthylène)]bis[amino-3 propionamide],
l'acide [dithiobis(éthanediylimino-2,1)] -5,5'bis [amino-4 oxo-5-pentanoïque],
le N,N'-[dithiobis(triméthylène)]bis[amino-2 acétamide],
le N,N'-[dithiobis(pentaméthylène)]bis [amino-2 acétamide],
le N,N'-[dithiobis(pentaméthylène)]bis [amino-4 butanamide],

le N,N'- [dithiobis(éthyloxy-2éthyl)] bis[amino-2 acétamide], et
le N,N'-(dithiodiéthanediyl-2,1) bis [amino-6 hexanamide].

Dans les compositions auto-neutralisantes le disulfure est généralement présent en un rapport molaire de 0,5 à 2,5 et de préférence de 1 à 2 par rapport au composé de formule générale (I) ou de ses sels (voir brevet US 3,768,490).

Les disulfures de formule générale (III) sont obtenus par oxydation des composés de formule générale (I) soit à l'air soit en utilisant des oxydants connus comme par exemple l'eau oxygénée en présence éventuellement de sels métalliques tels que par exemple les sels ferreux.

La présente invention a également pour objet à titre de produits industriels nouveaux les amino-mercaptoalkylamides répondant à la formule générale (IV) suivante :

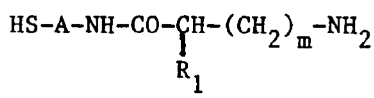 (IV)

dans laquelle :
A, $R_1$ et m ont les mêmes significations que données ci-dessus pour la formule générale (I) à l'exclusion des composés dans lesquels :

(i) A représente $-(CH_2)_2-$ et m est 0 ou 1, et $R_1$ représente un atome d'hydrogène, un radical alkyle, linéaire ou ramifié, ayant de 1 à 5 atomes de carbone, un radical benzyle ou un radical carboxy-2 éthyle, et

(ii) A représente $-(CH_2)_3-$ et m est 1, et $R_1$ représente un atome d'hydrogène,
et les sels desdits composés de formule (IV).

Les composés de formule (IV) sont préparés suivant les méthodes mentionnées pour la préparation des composés de formule (I).

Parmi les amino-mercaptoalkylamides de formule générale (IV) on peut notamment citer :
l'amino-2 N-(mercapto-3 propyl) acétamide,
l'amino-2 N-(mercapto-5 pentyl) acétamide,
l'amino-2 N-[mercapto-2 éthoxy)-2' éthyl] acétamide,
l'amino-2 N-(mercapto-3 propyl) propionamide,
l'amino-2 N-(mercapto-5 pentyl) propionamide,
l'amino-2 N-(mercapto-2 éthoxy-2' éthyl) propionamide,
l'amino-3 N-(mercapto-3 propyl) propionamide,
l'amino-3 N-(mercapto-5 pentyl) propionamide,
l'amino-3 [(mercapto-2 éthoxy)-2' éthyl] propionamide,
l'amino-4 N-(mercapto-2 éthyl) butanamide,
l'amino-4 N-(mercapto-3 propyl) butanamide,
l'amino-6 N-(mercapto-5 pentyl) butanamide,
l'amino-4 [(mercapto-2 éthoxy)-2'éthyl] butanamide, et
l'amino-6 N-(mercapto-2 éthyl) hexanamide,
ainsi que leurs chlorhydrates.

La présente invention a par ailleurs pour objet les nouveaux disulfures de formule générale (V) suivante :

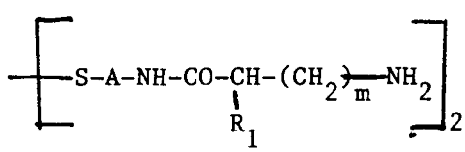 (V)

dans laquelle :
A, $R_1$ et m ont les mêmes significations que celles données ci-dessus pour la formule générale (IV).

Parmi les disulfures de formule (IV) on peut notamment citer :
le N,N'- [dithiobis(triméthylène)] bis [amino-2 acétamide],
le N,N'- [dithiobis(pentaméthylène)] bis [amino-2 acétamide],

le N,N'- [dithiobis(éthyloxy-2 éthyl)] bis [amino-2 acétamide],
le N,N'- [dithiodiéthanediyl-2,1] bis [amino-6 hexanamide],
le N,N'- [dithiodiéthanediyl-2,1] bis [amino-4 butanamide],
le N,N'- [dithiobis(triméthylène)] bis [amino-4 butanamide],
le N,N'- [dithiobis(pentaméthylène)] bis [amino-4 butanamide],
le N,N'- [dithiobis(triméthylène)] bis [amino-2 propionamide],
le N,N'- [dithiobis(triméthylène)] bis [amino-3 propionamide],
le N,N'- [dithiobis(pentaméthylène)] bis [amino-3 propionamide] ,et
le N,N'- [dithiobis(éthyloxy-2 éthyl)] bis [amino-3 propionamide].

La présente invention a en outre pour objet à titre de produits nouveaux les thioesters répondant à la formule générale (VI) suivante :

$$H_2N-A-S-CO-\underset{\substack{|\\R_1}}{CH}-(CH_2)_m-NH_2 \text{ , } 2HX \qquad (VI)$$

dans laquelle :
A, X, $R_1$ et m ont les mêmes significations que celles données ci-dessus pour la formule générale (II), à l'exclusion des composés dans lesquels :

(i) A représente $-(CH_2)_2-$ , m est 0 ou 1 et $R_1$ représente un atome d'hydrogène,

(ii) A représente $-(CH_2)_2-$ , m est 0, $R_1$ représente un radical méthyle, éthyle, isopropyle, isobutyle, méthylthioéthyle ou benzyle.

Le thioester ou A = $-CH_2)_2-$, m = 1 et $R_1$ = H est déjà décrit : Chemical Abstracts, 83 no. 21, 24-11-75, page 511, résumé no. 178345 t et JP=A-75-62931.

Les composés de formule générale (VI) sont obtenus par réaction d'un aminothiol (1) avec halogénure d'acide d'amino-acide (2) à une température comprise entre 40 et 110°C en présence ou non d'un solvant organique inerte tels que par exemple le dichlorométhane, le dichloro-1,2 éthane, le chloroforme, le tétrachlorure de carbone, l'acétonitrile, le toluène, le dioxane, le tétrahydrofuranne, le diméthoxy-1,2 éthane, la N-méthyl-pyrrolidone, la diméthylacétamide ou le diméthylformamide ou d'un mélange de ces solvants selon la réaction suivante :

$$\underset{(1)}{HX;\ H_2N-A-SH} + \underset{(2)}{X-CO-\underset{\substack{|\\R_1}}{CH}-(CH_2)_m-NH_2,\ HX}$$

$$\xrightarrow[-HX]{} H_2N-A-S-CO-\underset{\substack{|\\R_1}}{CH}-(CH_2)_m-NH_2 \text{ , } 2HX \qquad (VI)$$

Les aminothiols et les halogénures d'acides mis en oeuvre sont préparés selon les méthodes classiques de synthèse de ces composés.

Parmi les thioesters de formule générale (VI), on peut notamment citer :
le dichlorhydrate du thioglycinate d'amino-3 propyle,
le dichlorhydrate du thioglycinate d'amino-5 pentyle,
le dichlorhydrate du thioglycinate d'amino-2 éthoxy-2'éthyle,
le dichlorhydrate de l'amino-4 thiobutyrate d'amino-2 éthyle,
le dichlorhydrate de l'amino-4 thiobutyrate d'amino-3 propyle,
le dichlorhydrate de l'amino-4 thiobutyrate d'amino-5 pentyle,
le dichlorhydrate de l'amino-4 thiobutyrate d'(amino-2 éthoxy)-2'éthyle,

le dichlorhydrate de l'amino-6 thiocaproate d'amino-2 éthyle,
le dichlorhydrate du thioalaninate d'amino-3 propyle,
le dichlorhydrate du thioalaninate d'amino-5 pentyle, et
le dichlorhydrate du thioalaninate d'(amino-2 éthoxy)-2'éthyle.

La présente invention a également pour objet un procédé de déformation permanente des cheveux consistant, dans une première étape, à réduire les liaisons disulfures de la kératine par application, pendant environ 5 à 60 min, d'une composition réductrice telle que définie ci-dessus puis dans une seconde étape à reformer lesdites liaisons par application d'une composition oxydante ou éventuellement en laissant agir l'oxygène de l'air.

La présente invention a également pour objet un procédé d'ondulation des cheveux dans lequel on applique une composition réductrice telle que définie ci-dessus sur des cheveux mouillés préalablement enroulés sur des rouleaux ayant de 4 à 20 mm de diamètre, la composition pouvant éventuellement être appliquée au fur et à mesure de l'enroulage des cheveux ; on laisse ensuite agir la composition réductrice pendant un temps de 5 à 60 minutes, de préférence de 5 à 30 minutes, puis on rince abondamment après quoi on applique, sur les cheveux enroulés, une composition oxydante permettant de reformer les liaisons disulfures de la kératine pendant un temps de pose de 2 à 10 minutes. Après avoir enlevé les rouleaux, on rince abondamment la chevelure.

La composition d'oxydation ou oxydante est du type couramment utilisé et contient comme agent oxydant de l'eau oxygénée, un bromate alcalin, un persel, un polythionate ou un mélange de bromate alcalin et de persel. La concentration en eau oxygénée peut varier de 1 à 20 volumes et de préférence de 1 à 10, la concentration en bromate alcalin de 2 à 12 % et celle en persel de 0,1 à 15 % en poids par rapport au poids total de la composition oxydante. Le pH de la composition oxydante est généralement compris entre 2 et 10. Cette oxydation peut être effectuée immédiatement ou être différée.

La présente invention a également pour objet un procédé de défrisage ou de décrêpage des cheveux dans lequel on applique sur les cheveux une composition réductrice selon l'invention puis l'on soumet les cheveux à une déformation mécanique permettant de les fixer dans leur nouvelle forme, par une opération de lissage des cheveux avec un peigne à larges dents, avec le dos d'un peigne ou à la main. Après un temps de pose de 5 à 60 minutes, en particulier de 5 à 30 minutes, on procède alors à un nouveau lissage puis on rince soigneusement et on applique une composition oxydante ou fixatrice telle que définie ci-dessus, que l'on laisse agir pendant environ 2 à 10 minutes puis on rince abondamment les cheveux.

On va maintenant donner à titre d'illustration et sans aucun caractère limitatif plusieurs exemples de préparation des composés selon l'invention ainsi que des exemples de compositions réductrices selon l'invention et leur utilisation dans un procédé de déformation permanente des cheveux.

## EXEMPLES DE PREPARATION

### EXEMPLE 1 : Préparation du chlorhydrate d'amino-2 N-(mercapto-2 éthyl)propionamide

(a) Dichlorhydrate du thioalaninate d'amino-2 éthyle

A une suspension de 11,4 g (0,1 mole) de chlorhydrate de cystéamine dans 350 $cm^3$ d'acétonitrile anhydre, on ajoute, sous atmosphère inerte, 14,4 g de chlorhydrate du chlorure d'acide de la DL-alanine puis chauffe à 80°C sous agitation. Après deux heures, la réaction est complète (fin du dégagement d'acide chlorhydrique). On refroidit à température ambiante, essore le thioester formé et recristallise dans le méthanol. On obtient ainsi, après séchage sous vide à 50°C, 13,8 g de dichlorhydrate du thioalaninate d'amino-2 éthyle sous la forme d'un solide blanc de point de fusion : 226°C.

Le spectre RMN$^1$H 80 MHz est conforme à la structure attendue.

Analyse élémentaire : $C_5H_{12}N_2OS$, 2HCl

| | C % | H % | N % | O % | S % | Cl % |
|---|---|---|---|---|---|---|
| Calc. | 27,16 | 6,38 | 12,67 | 7,23 | 14,50 | 32,06 |
| Tr. | 27,25 | 6,36 | 12,60 | 7,45 | 14,40 | 32,02 |

(b) A une suspension de 22,1 g de dichlorhydrate du thioalaninate d'amino-2 éthyle (0,1 mole), obtenu ci-dessus, dans 50 cm³ d'isopropanol, on ajoute goutte à goutte, sous atmosphère inerte, 20,4 cm³ (0,15 mole) de solution aqueuse d'ammoniaque à 2,5 %. La solution obtenue est évaporée à sec sous pression réduite. Le résidu est repris par 100 cm³ d'éthanol. Le chlorure d'ammonium insoluble est séparé par filtration sur verre fritté. Le filtrat est évaporé à sec et séché sous vide à 50°C. On obtient ainsi 17,5 g d'amino-2 N-(mercapto-2 éthyl) propionamide sous la forme d'un solide blanc de point de fusion : 141°C.
Le spectre RMN¹H 80MHz est conforme à la structure attendue.

Analyse élémentaire : $C_5H_{12}N_2OS$, HCl

| | C % | H % | N % | O % | S % | Cl % |
|---|---|---|---|---|---|---|
| Calc. | 32,52 | 7,09 | 15,17 | 8,66 | 17,36 | 19,2 |
| Tr. | 31,63 | 7,17 | 14,80 | 10,29 | 16,97 | 18,69 |

EXEMPLE 2 : Préparation du chlorhydrate d'amino-2 N-(mercapto-2 éthyl)acétamide

(a) Dichlorhydrate du thioglycinate d'amino-2 éthyle

A une suspension de 11,4 g (0,1 mole) de chlorhydrate de cystéamine dans 50 cm³ d'acétonitrile anhydre,on ajoute sous atmosphère inerte et sous agitation, 13 g (0,1 mole) de chlorhydrate du chlorure d'acide de la glycine puis chauffe trois heures à 75°C. Après refroidissement à température ambiante, on essore le thioester brut qui est purifié par deux lavages successifs dans 40 cm³ de méthanol. On obtient, après séchage sous vide à 50°C, 15,1 g de dichlorhydrate du thioglycinate d'amino-2 éthyle sous la forme d'un solide blanc se décomposant à 165°C. Le spectre RMN¹H 80 MHz est conforme à la structure attendue.

Analyse élémentaire : $C_4H_{10}N_2OS$, 2HCl

| | C % | H % | N % | O % | S % | Cl % |
|---|---|---|---|---|---|---|
| Calc. | 23,20 | 5,84 | 13,53 | 7,72 | 15,48 | 34,23 |
| Tr. | 23,37 | 5,93 | 13,32 | 7,96 | 15,42 | 33,99 |

(b) A une suspension de 30 g (0,145 mole) de dichlorhydrate du thioglycinate d'amino-2 éthyle, obtenu ci-dessus, on ajoute goutte à goutte en 15 minutes, à température ambiante sous atmosphère inerte et sous agitation, 152 cm³ de solution aqueuse d'ammoniaque à 5 %. La solution obtenue est évaporée à sec sous pression réduite. On ajoute 100 cm³ d'isopropanol au résidu et évapore à nouveau à sec puis ajoute 200 cm³ d'éthanol, agite à température ambiante pour disperser finement et essore sur verre fritté le chlorure d'ammonium. Le filtrat est évaporé à sec. Le solide blanc obtenu (23,3 g) est recristallisé dans l'isopropanol. Après essorage et séchage sous vide, on obtient 16,4 g de chlorhydrate d'amino-2 N-(mercapto-2 éthyl)acétamide sous la forme d'un solide blanc de point de fusion : 122°C.
Les spectres RMN¹H 250 MHz et 13C sont conformes à la structure attendue.

Analyse élémentaire : $C_4H_{10}N_2OS$, HCl

| | C % | H % | N % | O % | S % | Cl % |
|---|---|---|---|---|---|---|
| Calc. | 28,15 | 6,50 | 16,41 | 9,37 | 18,79 | 20,77 |
| Tr. | 27,74 | 6,57 | 16,26 | 10,33 | 18,41 | 20,46 |

EXEMPLE 3 : Préparation du dichlorhydrate de l'amino-4 thiobutyrate d'amino-2 éthyle

A une suspension de 11,4 g (0,1 mole) de chlorhydrate de cystéamine dans 40 cm³ d'acétonitrile anhydre,

on ajoute, sous atmosphère inerte et sous agitation, 15,8 g (0,1 mole) de chlorhydrate du chlorure d'acide de l'acide amino-4 butyrique et chauffe 5 heures à 80°C. Après refroidissement à température ambiante, le solide blanc est essoré sur verre fritté puis recristallisé dans un mélange éthanol/méthanol 50/50. Après séchage sous vide à 50°C, on obtient 17,6 g de dichlorhydrate de l'amino-4 thiobutyrate d'amino-2 éthyle sous la forme d'un solide blanc de point de fusion : 186°C.

Le spectre RMN$^1$H 80MHz est conforme à la structure attendue.

Analyse élémentaire : $C_6H_{14}N_2OS$, 2HCl.

| | C % | H % | N % | O % | S % | Cl % |
|---|---|---|---|---|---|---|
| Calc. | 30,64 | 6,86 | 11,91 | 6,80 | 13,63 | 30,15 |
| Tr. | 29,88 | 6,92 | 11,59 | 7,46 | 13,40 | 29,33 |

EXEMPLE 4 : Préparation du dichlorhydrate de l'amino-6 thiocaproate d'amino-2 éthyle

Ce composé est préparé selon le même mode opératoire qu'à l'exemple 3 mais à partir de 18,6 g (0,1 mole) de chlorhydrate du chlorure d'acide de l'acide amino-6 caproïque. Après chauffage 4 heures à 80°C, on refroidit, essore et recristallise dans un mélange éthanol/méthanol 70/30. Après séchage sous vide à 50°C, on obtient 15,8 g de dichlorhydrate de l'amino-6 thiocaproate d'amino-2 éthyle sous la forme d'un solide blanc de point de fusion : 181°C.

Le spectre RMN$^1$H 80MHz est conforme à la structure attendue.

Analyse élémentaire : $C_8H_{18}N_2OS$, 2HCl.

| | C % | H % | N % | O % | S % | Cl % |
|---|---|---|---|---|---|---|
| Calc. | 36,50 | 7,66 | 10,64 | 6,08 | 12,18 | 26,94 |
| Tr. | 36,48 | 7,22 | 11,38 | 6,44 | 12,70 | 26,72 |

EXEMPLE 5 : Préparation du N,N'-(dithiodiéthanediyl-2,1) bis [amino-2 acétamide]

A une solution de 50,5 g (0,29 mole) de chlorhydrate d'amino-2 N-(mercapto-2 éthyl)acétamide, obtenu à l'exemple 2, dans 500 cm$^3$ d'éthanol absolu, on ajoute goutte à goutte 15,2 cm$^3$ (0,15 mole) d'eau oxygénée à 110 volumes en maintenant la température inférieure à 25°C. Après 24 heures d'agitation, la cristallisation du disulfure est complète. On essore sur verre fritté, lave par 100 cm$^3$ d'éthanol absolu et sèche sous vide à 60°C. On obtient 43,1 g de cristaux blancs de N,N'-(dithiodiéthanediyl-2,1) bis [amino-2 acétamide] de point de fusion : 168-170°C.

Le spectre RMN$^1$H 80 MHz est conforme à la structure attendue.

Analyse élémentaire : $C_8H_{18}N_4O_2S_2$, 2HCl.

| | C % | H % | N % | O % | S % | Cl % |
|---|---|---|---|---|---|---|
| Calc. | 28,32 | 5,94 | 16,51 | 9,43 | 18,90 | 20,90 |
| Tr. | 28,04 | 6,09 | 16,23 | 10,45 | 18,69 | 20,64 |

EXEMPLE 6 : Préparation du dichlorhydrate d'amino-4 thiobutyrate d'amino-3 propyle

Une suspension de 12,8 g (0,1 mole) de chlorhydrate de mercapto-3 propylamine et de 11,58 g (0,1 mole) de chlorhydrate du chlorure d'acide de l'acide amino-4 butyrique dans 75 cm$^3$ d'acétonitrile anhydre est agitée

sous atmosphère inerte et est chauffée 4 heures au reflux.

Après refroidissement à température ambiante, le thioester brut est essoré, lavé par de l'éthanol glacé puis recristalisé dans un mélange Ethanol/Méthanol (75/25).

Après séchage sous vide à 50°C, on obtient 17,5 g de dichlorhydrate d'amino-4 thiobutyrate d'amino-3 propyle sous la forme d'un solide blanc de point de fusion 205°C.

Le spectre RMN$^1$H 80 MHz est conforme à la structure attendue.

EXEMPLE 7 : Préparation du dichlorhydrate du thioglycinate d'(amino-2 éthoxy)-2 éthyle

Une suspension de 15,7 g (0,1 mole) de chlorhydrate de (mercapto-2 éthoxy)-2' éthylamine et de 13 g (0,1 mole) de chlorhydrate du chlorure d'acide de la glycine dans 120 cm$^3$ d'acétonitrile anhydre, est chauffée sous atmosphère inerte et sous agitation à 30°C pendant 30mn, puis au reflux pendant 1 h 30.

Après refroidissement à température ambiante, le thioester brut est essoré, puis recristallisé dans environ 250 cm$^3$ d'éthanol.

Après séchage sous vide à 40°C, on obtient 10 g de dichlorhydrate du thioglycinate d'(amino-2 éthoxy)-2'éthyle sous la forme d'un solide blanc de point de fusion 145°C (décomposition).

Le spectre RMN$^1$H 80 MHz est conforme à la structure attendue.

EXEMPLE 8 : Préparation du dichlorhydrate du thioglycinate d'amino-5 pentyle

Une suspension de 15,55 g (0,1 mole) de chlorhydrate de mercapto-5 pentylamine et de 13 g (0,1 mole) de chlorhydrate du chlorure d'acide de la glycine dans 60 cm$^3$ d'acétonitrile anhydre, est chauffée sous atmosphère inerte, et sous agitation 3 heures au reflux.

Après refroidissement à température ambiante, le thioester brut est essoré, lavé avec de l'isopropanol puis recristalisé dans un mélange d'éthanol-méthanol.

Après séchage sous vide à 30°C, on obtient 18,6 g de dichlorhydrate du thioglycinate d'amino-5 pentyle sous la forme d'un solide blanc de point de fusion 230°C (décomposition).

Le spectre RMN$^1$H est conforme à la structure attendue.

EXEMPLES DE COMPOSITION

EXEMPLE A

On prépare selon l'invention une composition réductrice de déformation permanente des cheveux en procédant au mélange des ingrédients suivants :

A. COMPOSITION REDUCTRICE

| | |
|---|---|
| Chlorhydrate d'amino-2 N-(mercapto-2 éthyl)acétamide............. | 17 g |
| Monoéthanolamine q.s.p. pH 8,5 | |
| Chlorure d'oléocétyl diméthyl hydroxyéthyl ammonium............. | 0,3 g |
| Conservateur.................................................... | 0,2 g |
| Parfum.......................................................... | 0,8 g |
| Eau déminéralisée..............q.s.p.......................... | 100 g |

Cette composition est appliquée sur des cheveux mouillés préalablement enroulés sur des rouleaux. Après avoir laissé agir la composition 15 minutes, on rince abondamment à l'eau puis on applique la composition oxydante suivante :

B. COMPOSITION OXYDANTE

Eau oxygénée.................................................... 1,5 g
Lauryl éther sulfate de sodium oxyéthyléné avec 2 moles
d'oxyde d'éthylène.............................................. 3,75 g
Acide citrique.................................................. 0,5 g
Hydrogénophosphate de sodium.................................... 0,5 g
Parfum.......................................................... 0,3 g
Eau déminéralisée..............q.s.p.......................... 100 g

On laisse agir la composition oxydante pendant environ 5 minutes, puis on enlève les rouleaux, et rince abondamment la chevelure à l'eau. Après séchage sous casque, les cheveux présentent de belles boucles.

Selon le même mode de réalisation qu'à l'exemple A, on a procédé à une déformation permanente des cheveux à l'aide des compositions réductrices et oxydantes des exemples B à P suivants :

EXEMPLE B

A. COMPOSITION REDUCTRICE

Chlorhydrate d'amino-2 N(mercapto-2 éthyl)acétamide............. 21 g
Acide éthylène diamine tétracétique............................. 0,2 g
Triéthanolamine q.s.p......pH 6,8
Oxyde de Lauramine vendu sous la dénomination de
"Aromox DMMCD/W" par la Société AKZO............................ 2,15 g
Parfum.......................................................... 0,6 g
Eau déminéralisée q.s.p......................................... 100 g

B. COMPOSITION OXYDANTE

Eau oxygénée.................................................... 2,0 g
Chlorure d'oléocétyl diméthyl hydroxyéthyl ammonium............. 0,3 g
Acide phosphorique.............................................. 0,5 g
p-éthoxyacétanilide (phénacétine)............................... 0,1 g
Hydrolysat de protéines......................................... 0,5 g
Parfum.......................................................... 0,5 g
Eau déminéralisée....q.s.p...................................... 100 g

EXEMPLE C

A. COMPOSITION REDUCTRICE

| | |
|---|---|
| Dichlorhydrate du thioglycinate d'amino-2 éthyle................ | 10 g |
| Solution ammoniacale à 20 % $NH_3$.............................. | 4,1 g |
| Monoéthanolamine q.s.p........ pH 8,0 | |
| Hydrogénocarbonate d'ammonium................................... | 2,0 g |
| Cocoamidopropylbétaïne vendu sous la dénomination de "Tegobetaïne HS" par la Société GOLDSCHMIDT..................... | 0,9 g |
| Eau déminéralisée... q.s.p....................................... | 100 g |

B. COMPOSITION OXYDANTE

| | |
|---|---|
| Eau oxygénée..................................................... | 2,5 g |
| Stannate de sodium............................................... | 0,03 g |
| Chlorure de N,N diméthyl N-2 propényl-2 propéne-1 ammonium (polyquaternium-6) vendu sous la dénomination de "Merquat 100" par la Société MERCK.......................... | 1,25 g |
| Acide citrique................................................... | 0,6 g |
| Parfum........................................................... | 0,5 g |
| Eau déminéralisée...q.s.p........................................ | 100 g |

EXEMPLE D

A. COMPOSITION REDUCTRICE

| | |
|---|---|
| Dichlorhydrate du thioglycinate d'amino-2 éthyle................ | 15 g |
| N,N'-(dithiodiéthanediyl-2,1) bis amino-2 acétamide............ | 4,5 g |
| Sel pentasodique de l'acide diéthylène triamine pentacétique.... | 0,2 g |
| Monoéthanolamine..q.s.p....... pH 8,9 | |
| Huile de castor hydrogénée oxyéthyléné avec 60 moles d'oxyde d'éthylène vendu sous la dénomination de "NIKKOL HCO 60" par la Société NIKKO CHEMICAL.................. | 4 g |
| Chlorure de N,N diméthyl N-2 propenyl 2 propène 1 ammonium (polyquaternium 7) vendu sous la dénomination de "Merquat 550" par la Société MERCK............................. | 3,8 g |
| Parfum........................................................... | 0,3 g |
| Eau déminéralisée q.s.p.......................................... | 100 g |

B. COMPOSITION OXYDANTE

| | |
|---|---|
| Eau oxygénée (200 volumes) | 4,8 g |
| Stabilisant | 0,1 g |
| D. Panthenol | 1,0 g |
| α-diméthyl-4'α-(méthyl-4 pentényl-3) cyclohexène-3 méthanol (Bisabolol) vendu sous le nom commercial de "Dragosantol 2/012681" par la Société DRAGOCO | 0,3 g |
| Oxyde de lauryl diméthyl amine | 0,7 g |
| Parfum | 0,4 g |
| Acide lactique q.s.p......pH 3,0 | |
| Eau déminéralisée q.s.p | 100 g |

EXEMPLE E

A. COMPOSITION REDUCTRICE

| | |
|---|---|
| Dichlorhydrate du thioglycinate d'amino-2 éthyle | 5 g |
| Dichlorhydrate de l'amino-4 thiobutyrate d'amino-2 éthyle | 6,5 g |
| Solution ammoniacale à 20 % | 4,4 g |
| Monoéthanolamine q.s.p....pH 7,5 | |
| Oléyl éther oxyéthyléné avec 20 moles d'oxyde d'éthylène | 6,0 g |
| Lauryl éther oxyéthyléné avec 23 moles d'oxyde d'éthylène | 1,0 g |
| Sel pentasodique de l'acide diéthylène triamine pentacétique | 0,5 g |
| Parfum | 0,3 g |
| Eau déminéralisée q.s.p | 100 g |

B. COMPOSITION OXYDANTE

| | |
|---|---|
| Eau oxygénée | 1,5 g |
| Lauryl éther sulfate de sodium oxyéthyléné avec 2 moles d'oxyde d'éthylène | 3,75 g |
| Acide citrique | 0,5 g |
| Hydrogénophosphate de sodium | 0,5 g |
| Parfum | 0,3 g |
| Eau déminéralisée q.s.p | 100 g |

EXEMPLE F

A. COMPOSITION REDUCTRICE

| | |
|---|---|
| Dichlorhydrate du thioglycinate d'amino-2 éthyle................ | 4 g |
| Dichlorhydrate du thioalaninate d'amino-2 éthyle................ | 3 g |
| Chlorhydrate d'amino-2 N-(mercapto-2 éthyl) propionamide........ | 2,5 g |
| Monoéthanolamine q.s.p.........pH 8,7 | |
| Chlorure de cétrimonium vendu sous la dénomination de "Dehyquart A" par la Société HENKEL.......................... | 1 g |
| Parfum.......................................................... | 0,7 g |
| Eau déminéralisée q.s.p......................................... | 100 g |

B. COMPOSITION OXYDANTE

| | |
|---|---|
| Bromate de sodium............................................... | 8,0 g |
| Triéthanolamine q.s.p.............pH 8,0 | |
| Phosphate monosodique hydraté (12 $H_2O$)........................ | 0,3 g |
| Phosphate trisodique hydraté (2 $H_2O$).......................... | 0,5 g |
| Cocoamidopropylbétaïne vendu sous le nom commercial de "Tegobetaïne HS" par la Société GOLDSCHMIDT................. | 1,0 g |
| Parfum.......................................................... | 0,4 g |
| Eau déminéralisée q.s.p......................................... | 100 g |

EXEMPLE G

A. COMPOSITION REDUCTRICE

| | |
|---|---|
| Dichlorhydrate du thioglycinate d'amino-2 éthyle................ | 3,0 g |
| L-cystéine...................................................... | 5,0 g |
| Monoéthanolamine q.s.p...... pH 9,3 | |
| Ester stéarique polyéthyléné avec 8 moles d'oxyde d'éthylène vendu sous la dénomination de "Myrj 45" par la Société ICI.......................................... | 1 g |
| Conservateur.................................................... | 0,4 g |
| Parfum.......................................................... | 0,3 g |
| Eau déminéralisée q.s.p......................................... | 100 g |

B. COMPOSITION OXYDANTE

| | |
|---|---|
| Eau oxygénée | 2,5 g |
| Stannate de sodium | 0,02 g |
| Lauryl sulfate d'ammonium | 1,5 g |
| Hydrolysat de protéines | 0,6 g |
| Acide citrique | 0,5 g |
| Parfum | 0,4 g |
| Eau déminéralisée q.s.p | 100 g |

## EXEMPLE H

A. COMPOSITION REDUCTRICE

| | |
|---|---|
| Dichlorhydrate du thioglycinate d'amino-2 éthyle | 2,0 g |
| N-acétylcystéamine | 6,0 g |
| Solution ammoniacale q.s.p.........pH 8,0 | |
| Hydrogénocarbonate d'ammonium | 2,0 g |
| Chlorure de N,N diméthyl N-2 propényl-2 propéne-1 ammonium (polyquaternium-6) vendu sous la dénomination de "Merquat 100" par la Société MERCK | 2,5 g |
| Hydrolysat de collagène | 0,5 g |
| Chlorure d'oléocétyl diméthyl hydroxyéthyl ammonium | 1,0 g |
| Parfum | 0,8 g |
| Eau déminéralisée q.s.p | 100 g |

B. COMPOSITION OXYDANTE

| | |
|---|---|
| Eau oxygénée (200 volumes) | 4,8 g |
| Stabilisant | 0,1 g |
| D. Panthenol | 1,0 g |
| α-diméthyl-4'α-(méthyl-4 pentenyl-3) cyclohexène-3 méthanol (Bisabolol) vendu sous la dénomination de "Dragosantol 2/012681" par la Société DRAGOCO | 0,3 g |
| Oxyde de lauryl diméthyl amine | 0,7 g |
| Parfum | 0,4 g |
| Acide lactique q.s.p...............pH 3,0 | |
| Eau déminéralisée q.s.p | 100 g |

EXEMPLE I

A. COMPOSITION REDUCTRICE

| | |
|---|---|
| Dichlorhydrate du thioglycinate d'amino-2 éthyle | 5,5 g |
| Dichlorhydrate de l'amino-6 thiocaproate d'amino-2 éthyle | 7,3 g |
| Ammoniaque (20 %) | 5,1 g |
| Monoéthanolamine q.s.p. pH 8,3 | |
| Cocoamidopropylbétaïne vendu sous la dénomination de "Tegobetaïne HS" par la Société GOLDSCHMIDT | 0,9 g |
| Copolymère vinyl pyrrolidone/chlorure de méthacrylamidopropyl triméthyl ammonium à 20 % dans l'eau vendu sous la dénomination de "Gafquat HS 100" par la Société GAF | 1,0 g |
| Conservateur | 0,2 g |
| Parfum | 0,7 g |
| Eau déminéralisée q.s.p | 100 g |

B. COMPOSITION OXYDANTE

| | |
|---|---|
| Eau oxygénée | 2,0 g |
| Chlorure d'oléocétyl diméthyl hydroxyéthyl ammonium | 0,3 g |
| Acide phosphorique | 0,5 g |
| p-éthoxyacétanilide (Phénacétine) | 0,1 g |
| Hydrolysat de protéines | 0,8 g |
| Parfum | 0,5 g |
| Eau déminéralisée q.s.p | 100 g |

EXEMPLE J

A. COMPOSITION REDUCTRICE

| | |
|---|---|
| Dichlorhydrate du thioglycinate d'amino-2 éthyle | 1,5 g |
| Cystéamine, HCl | 5,2 g |
| Monoéthanolamine q.s.p. pH 9,5 | |
| Chlorure de cétyl triméthyl ammonium | 1,0 g |
| Parfum | 0,6 g |
| Conservateur | 0,15 g |
| Eau déminéralisée q.s.p | 100 g |

B. COMPOSITION OXYDANTE

| | |
|---|---|
| Bromate de sodium | 8 g |
| Triéthanolamine q.s.p. pH 8,0 | |
| Phosphate monosodique hydraté (12 $H_2O$) | 0,3 g |
| Phosphate trisodique hydraté (2 $H_2O$) | 0,5 g |
| Cocoamidopropylbétaïne vendu sous la dénomination "Tegobétaïne HS" par la Société GOLDSCHMIDT | 1,0 g |
| Parfum | 0,4 g |
| Eau déminéralisée q.s.p | 100 g |

EXEMPLE K

A. COMPOSITION REDUCTRICE

| | |
|---|---|
| Dichlorhydrate du thioglycinate d'amino-2 éthyle | 5,5 g |
| Dichlorhydrate de l'amino-3 thiopropionate d'amino-2 éthyle | 7,3 g |
| Ammoniaque (20 %) | 5,1 g |
| Monoéthanolamine q.s.p. pH 8,3 | |
| Cocoamidopropylbétaïne vendu sous la dénomination de "Tegobetaïne HS" par la Société GOLDSCHMIDT | 0,9 g |
| Copolymère vinyl pyrrolidone/chlorure de méthacrylamidopropyl triméthyl ammonium à 20 % dans l'eau vendu sous la dénomination de "Gafquat HS 100" par la Société GAF | 1,0 g |
| Conservateur | 0,2 g |
| Parfum | 0,7 g |
| Eau déminéralisée q.s.p | 100 g |

B. COMPOSITION OXYDANTE

| | |
|---|---|
| Eau oxygénée | 2,0 g |
| Chlorure d'oléocétyl diméthyl hydroxyéthyl ammonium | 0,3 g |
| Acide phosphorique | 0,5 g |
| p-éthoxyacétanilide (Phénacétine) | 0,1 g |
| Hydrolysat de protéines | 0,8 g |
| Parfum | 0,5 g |
| Eau déminéralisée q.s.p | 100 g |

EXEMPLE L

A. COMPOSITION REDUCTRICE :

Amino-3 N-(mercapto-2 éthyl) propionamide (Aléthéine).......... 18 g
Chlorure d'oléocétyldiméthylhydroxyéthylammonium............... 1,3 g
Sel pentasodique de l'acide diéthylénetriaminopentacétique...... 0,2 g
Ammoniaque à 20 % q.s .........pH = 8,5
Eau déminéralisée q.s.p......................................... 100 g

B. COMPOSITION OXYDANTE :

Eau oxygénée à 100 vol......................................... 4,8 g
Sulfate d'hydoxy-8 quinolène................................... 0,0125 g
p-éthoxyacétamilide (phénacétine).............................. 0,05 g
Chlorure d'oléocétyldiméthylhydroxyéthylammonium............... 0,3 g
Acide citrique q.s.p..........pH = 3,0
Eau déminéralisée q.s.p......................................... 100 g

Dans cet exemple la composition réductrice peut-être obtenue, au moment de l'emploi, à partir du précurseur thioester de l'aléthéine.

Selon ce mode de réalisation, on mélange 22,1 g du dichlorhydrate de l'amino-3 thiopropionate d'amino-2 éthyle sous forme de poudre à une solution aqueuse basique ayant la composition suivante :

Chlorure d'oléocétyldiméthylhydroxyéthylammonium............... 1,3 g
Sel pentasodique de l'acide diéthylénetriaminopentacétique...... 0,2 g
Ammoniaque q.s.p. pH = 8,5
Eau déminéralisée q.s.p......................................... 100 g

EXEMPLE M :

A. COMPOSITION REDUCTRICE

Dichlorhydrate de l'amino-3 thiopropionate d'amino-2 éthyle..... 18,4 g
Ammoniaque (solution à 20 %) q.s.p......pH = 6,9
Mélange copolymère vinyl pyrrolidone/méthacrylate de diméthyl aminoéthyle quaternisé par du sulfate de diméthyle vendu sous le nom commercial de "Gafquat 755" par la Société GAF............. 5,0 g
Cocoamido éthyl (N-hydroxyéthyl, N-carboxyméthyl) glycinate de sodium vendu sous le nom commercial de "Miranol C 2M concentré NP" par la Société MIRANOL................................. 1,3 g
Parfum......................................................... 0,5 g
Conservateur................................................... 0,2 g
Eau déminéralisée..q.s.p........................................ 100 g

B. COMPOSITION OXYDANTE

| | |
|---|---|
| Eau oxygénée | 2,5 g |
| Stabilisants | 0,1 g |
| Oxyde de lauramine vendu sous le nom commercial de "Aromex DMMCD/W" par la Société AKZO | 2,15 g |
| Parfum | 0,4 g |
| Acide lactique qsp pH = 3,0 | |
| Eau déminéralisée q.s.p | 100 g |

EXEMPLE N

A. COMPOSITION REDUCTRICE

| | |
|---|---|
| Dichlorhydrate de l'amino-3 thiopropionate d'amino-2 éthyle | 16 g |
| Dichlorhydrate du N,N'-(dithiodiéthanediyl-2,1) bis amino-3 propionamide | 7,5 g |
| Ammoniaque (solution à 20 %) qsp pH = 7,0 | |
| Monoétholamine | 3,3 g |
| Parfum | 0,7 g |
| Conservateur | 0,2 g |
| Chlorure d'oléocétyl diméthyl hydroxyéthyl ammonium | 0,3 g |
| Eau déminéralisée q.s.p | 100 g |

B. COMPOSITION OXYDANTE

| | |
|---|---|
| Bromate de sodium | 8,0 g |
| Triéthanolamine qsp pH = 8,0 | |
| Phosphate monosodique hydraté (12 $H_2O$) | 0,3 g |
| Phosphate trisodique hydraté (2 $H_2O$) | 0,5 g |
| Cocoamidopropyl bétaine vendu sous le nom commercial de "Tegobetaine HS" par la Société GOLDSMIDT | 2,8 g |
| Eau déminéralisée qsp | 100 g |

EXEMPLE O

A. COMPOSITION REDUCTRICE

| | |
|---|---|
| Dichlorhydrate de l'amino-3 thiopropionate d'ammino-2 éthyle... | 3 g |
| L-cystéine.................................................. | 5 g |
| Monoéthanolamine q.s.p......pH = 9,3 | |
| Ester stéarique polyéthyléné avec 8 moles d'oxyde d'éthylène vendu sous la dénomination de "Myrj 45" par la Société ICI..... | 1 g |
| Conservateur ............................................. | 0,4 g |
| Parfum.................................................... | 0,3 g |
| Eau déminéralisée........q.s.p................................ | 100 g |

B. COMPOSITION OXYDANTE

| | |
|---|---|
| Eau oxygénée.............................................. | 2,5 g |
| Stannate de sodium........................................ | 0,02 g |
| Lauryl sulfate d'ammonium................................. | 1,5 g |
| Hydrolysat de protéines................................... | 0,6 g |
| Acide citrique............................................ | 0,5 g |
| Parfum.................................................... | 0,4 g |
| Eau déminéralisée.......q.s.p................................ | 100 g |

EXEMPLE P

A. COMPOSITION REDUCTRICE

| | |
|---|---|
| Dichlorhydrate de l'amino-3 thiopropionate d'amino-2 éthyle.. | 1,5 g |
| Cystéamine, HCl........................................... | 5,2 g |
| Monoéthanolamine q.s.p........pH = 9,5 | |
| Chlorure de cétyl triméthyl ammonium...................... | 1,0 g |
| Conservateur.............................................. | 0,15 g |
| Parfum.................................................... | 0,6 g |
| Eau déminéralisée.......q.s.p................................ | 100 g |

B. COMPOSITION OXYDANTE

| | |
|---|---|
| Bromate de sodium.............................................. | 8 g |
| Triéthanolamine..........................pH = 8,0 | |
| Phosphate monosodique hydraté (12 $H_2O$)..................... | 0,3 g |
| Phosphate trisodique hydraté (2 $H_2O$)....................... | 0,5 g |
| Cocoamidopropyl bétaine vendu sous la dénomination de "Tegobetaine HS" par la Société GOLDSCHMIDT.................. | 1,0 g |
| Parfum......................................................... | 0,4 g |
| Eau déminéralisée......q.s.p............................... | 100 g |

## Revendications

1. Composition cosmétique pour le premier temps d'une opération de déformation permanente des cheveux caractérisée par le fait qu'elle contient, dans un véhicule cosmétique approprié, en tant qu'agent réducteur, au moins un amino mercaptoalkylamide ayant la formule générale suivante :

$$HS\text{-}A\text{-}NH\text{-}CO\text{-}\underset{\displaystyle R_1}{\underset{|}{CH}}\text{-}(CH_2)_m\text{-}NH_2 \qquad (I)$$

dans laquelle :

A représente le radical divalent $-(CH_2)_n-$, n étant un nombre entier compris entre 2 et 5, ou le radical divalent $-(CH_2)_2\text{-}O\text{-}(CH_2)_2-$ m est 0 ou un nombre entier compris entre 1 et 4,

(i) lorsque m est O, $R_1$ représente un atome d'hydrogène, un radical méthyle, un radical éthyle, un radical isopropyle, un radical isobutyle, un radical méthyl-2-butyle, un radical benzyle, un radical amino-4 butyle, un radical guanidino-3 propyle, un radical méthylthio-2 éthyle, un radical carboxyméthyle ou un radical carboxy-2 éthyle,

(ii) lorsque m est un nombre entier de 1 à 4, $R_1$ représente un atome d'hydrogène ou un radical alkyle inférieur ayant de 1 à 5 atomes de carbone,

et les sels desdits composés de formule (I).

2. Composition selon la revendication 1, caractérisée par le fait que les sels, des composés de formule (I) sont choisis parmi les chlorhydrates, bromhydrates, citrates et oxalates.

3. Composition selon la revendication 1 ou 2, caractérisée par le fait que les composés sont choisis parmi :
   - l'amino-3 N-(mercapto-2 éthyl) propionamide ou aléthéine,
   - l'amino-2 N-(mercapto-2 éthyl) acétamide,
   - l'amino-2 N-(mercapto-3 propyl) acétamide,
   - l'amino-2 N-(mercapto-5 pentyl) acétamide,
   - l'amino-2 N-[(mercapto-2 éthoxy)-2'éthyl]acétamide,
   - l'amino-2 N-(mercapto-2 éthyl) propionamide,
   - l'amino-2 N-(mercapto-3 propyl) propionamide,
   - l'amino-2 N-(mercapto-5 pentyl) propionamide,
   - l'amino-2 N-(mercapto-2 éthoxy-2' éthyl) propionamide,

- l'amino-3 N-(mercapto-3 propyl) propionamide,
- l'amino-3 N-(mercapto-5 pentyl) propionamide,
- l'amino-3 N-[(mercapto-2 éthoxy)-2' éthyl] propionamide,
- l'amino-2 N-(mercapto-2 éthyl) méthyl-3 butanamide,
- l'amino-2 N-(mercapto-2 éthyl) méthyl-4 pentanamide,
- l'αamino N-(mercapto-2 éthyl) hydrocinnamide)
- l'acide amino-4[(mercapto-2 éthyl) amino]-5 oxo-5 pentanoïque,
- l'amino-4 N-(mercapto-2 éthyl) butanamide,
- l'amino-4 N-(mercapto-3 propyl) butanamide,
- l'amino-6 N-(mercapto-5 pentyl) butanamide,
- l'amino-4[N-(mercapto-2 éthoxyl)-2' éthyl)]butanamide, et
- l'amino-6 N-(mercapto-2 éthyl) hexanamide,

ainsi que leurs chlorhydrates.

**4.** Composition selon les revendications 1 à 3, caractérisée par le fait que le composé de formule (I) est formé in situ à partir de son précurseur thioester ayant la formule générale (II) suivante :

$$H_2N-A-S-CO-\underset{\displaystyle R_1}{\underset{|}{CH}}-(CH_2)_m-NH_2,\ 2HX \qquad (II)$$

dans laquelle :
A, $R_1$et m ont les mêmes significations que celles données à la revendication 1 pour la formule générale (I), et
X est Cl ou Br.

**5.** Composition selon l'une quelconque des revendications précédentes, caractérisée par le fait que le composé de formule (I) ou (II) est présent à une concentration comprise entre 2 et 30 % et de préférence entre 5 et 25 % en poids par rapport au poids total de la composition.

**6.** Composition selon l'une quelconque des revendications précédentes, caractérisée par le fait qu'elle présente un pH compris entre 4,5 et 11, et de préférence entre 6 et 10, obtenu à l'aide d'un agent alcalin choisi parmi l'ammoniaque, la monoéthanolamine, la diéthanolamine, la triéthanolamine, un carbonate ou un bicarbonate alcalin ou d'ammonium, un hydroxyde alcalin ou à l'aide d'un agent acidifiant choisi parmi l'acide chlorhydrique, l'acide acétique, l'acide lactique, l'acide oxalique ou l'acide borique.

**7.** Composition selon l'une quelconque des revendications précédentes, caractérisée par le fait qu'elle contient en outre au moins un autre agent réducteur choisi parmi : l'acide thioglycolique, le monothioglycolate de glycérol ou du glycol, la cystéamine et ses dérivés acylés $C_1$-$C_4$, la cystéine, la N-acétylcystéine, le N-(mercapto-2 éthyl) gluconamide, l'acide β-mercaptopropionique et ses dérivés, l'acide thiolactique, la pantéthéine, le thioglycérol, un sulfite et un bisulfite de métal alcalin ou alcalino-terreux.

**8.** Composition selon l'une quelconque des revendications précédentes, caractérisée par le fait qu'elle contient, en outre, au moins un polymère cationique, un agent adoucissant, un hydrolysat de protéines, une cire, un agent opacifiant, un parfum, un colorant, un agent tensio-actif non-ionique ou cationique, un alcool, un agent traitant ou encore un agent de pénétration.

**9.** Composition selon l'une quelconque des revendications précédentes, caractérisée par le fait qu'elle contient, en outre, au moins un disulfure, la composition étant du type auto-neutralisante.

**10.** Composition selon la revendication 9, caractérisée par le fait que le disulfure est l'acide dithioglycolique, le dithioglycérol, la cystamine, la N,N'-diacétyl-cystamine, la cystine ou la panthétine.

**11.** Composition selon la revendication 9, caractérisé par le fait que le disulfure correspond à la formule générale (III) suivante :

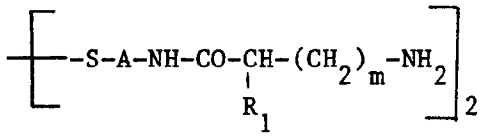

(III)

dans laquelle :
A, $R_1$ et m ont les mêmes significations que celles données à la revendication 1 pour la formule générale (I).

**12.** Composition selon la revendication 11, caractérisée par le fait que le disulfure de formule (III) est choisi parmi :

le N,N'-(dithiodiéthanediyl-2,1) bis [amino-3 propionamide],
le N,N'-(dithiodiéthanediyl-2,1) bis [amino-2 acétamide],
le N,N'-(dithiodiéthanediyl-2,1) bis [amino-2 propionamide],
le N,N'-[dithiobis (triméthylène)] bis [amino-2 propionamide],
le N,N'-(dithiodiéthanediyl-2,1)bis [α-amino benzène propionamide]
le N,N'-(dithiodiéthanediyl-2,1)bis [amino-2 méthyl-4 pentanamide],
le N,N'-(dithiodiéthanediyl-2,1)bis [amino-4 butanamide],
le N,N'-[dithiobis(triméthylène)]bis[amino-3 propionamide],
l'acide [dithiobis(éthanediylimino-2,1)] -5,5'bis [amino-4 oxo-5-pentanoïque],
le N,N'-[dithiobis(triméthylène)]bis[amino-2 acétamide],
le N,N'-[dithiobis(pentaméthylène)]bis [amino-2 acétamide],
le N,N'-[dithiobis(pentaméthylène)]bis [amino-4 butanamide],
le N,N-'[dithiobis(éthyloxy-2éthyl)] bis[amino-2 acétamide], et
le N,N'-(dithiodiéthanediyl-2,1) bis [amino-6 hexanamide].

**13.** Composition selon l'une quelconque des revendications 8 à 12, caractérisée par le fait que le disulfure est présent en une proportion molaire par rapport au composé de formule (I) allant de 0,5 à 2,5 et de préférence de 1 à 2.

**14.** Composés nouveaux, caractérisés par le fait qu'ils répondent à la formule générale (IV) suivante :

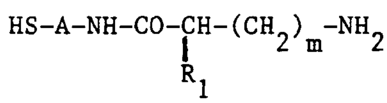

(IV)

dans laquelle :
A, $R_1$ et m ont les mêmes significations que celles données à la revendication 1 pour la formule générale (I) à l'exclusion des composés dans lesquels :

(i) A représente $-(CH_2)_2-$ et m est 0 ou 1, et $R_1$ représente un atome d'hydrogène, un radical alkyle, linéaire ou ramifié ayant de 1 à 5 atomes de carbone, un radical benzyle ou un radical carboxy-2 éthyle, et

(ii) A représente $-(CH_2)_3-$ et m est 1, et $R_1$ représente un atome d'hydrogène, et les sels desdits composés de formule (IV) ainsi que leurs disulfures correspondants.

**15.** Composés selon la revendication 14, caractérisés par le fait que les composés de formule (IV) sont choisis parmi :

l'amino-2 N-(mercapto-3 propyl) acétamide,
l'amino-2 N-(mercapto-5 pentyl) acétamide,
l'amino-2 N-[(mercapto-2 éthoxy)-2' éthyl] acétamide,
l'amino-2 N-(mercapto-3 propyl) propionamide,

l'amino-2 N-(mercapto-5 pentyl) propionamide,
l'amino-2 N-(mercapto-2 éthoxy-2' éthyl) propionamide,
l'amino-3 N-(mercapto-3 propyl) propionamide,
l'amino-3 N-(mercapto-5 pentyl) propionamide,
l'amino-3 N-[(mercapto-2 éthoxy)-2' éthyl] propionamide,
l'amino-4 N-(mercapto-2 éthyl) butanamide,
l'amino-4 N-(mercapto-3 propyl) butanamide,
l'amino-6 N-(mercapto-5 pentyl) butanamide,
l'amino-4 N-[(mercapto-2 éthoxy)-2'éthyl] butanamide, et
l'amino-6 N-(mercapto-2 éthyl) hexanamide,
ainsi que leurs chlorhydrates.

**16.** Composés selon la revendication 14, caractérisés par le fait que les disulfures sont choisis parmi :
le N,N'-[dithiobis(triméthylène)] bis [amino-2 acétamide],
le N,N'-[dithiobis(pentaméthylène)] bis [amino-2 acétamide],
le N,N'-[dithiobis(éthyloxy-2 éthyl)] bis [amino-2 acétamide],
le N,N'-[dithiodiéthanediyl-2,1] bis [amino-6 hexanamide],
le N,N'-[dithiodiéthanediyl-2,1] bis [amino-4 butanamide],
le N,N'-[dithiobis(triméthylène)] bis [amino-4 butanamide],
le N,N'-[dithiobis(pentaméthylène)] bis [amino-4 butanamide],
le N,N'-[dithiobis(triméthylène)] bis [amino-2 propionamide],
le N,N'-[dithiobis(triméthylène)] bis [amino-3 propionamide],
le N,N'-[dithiobis(pentaméthylène)] bis [amino-3 propionamide],et
le N,N'-[dithiobis(éthyloxy-2 éthyl)] bis [amino-3 propionamide].

**17.** Composés nouveaux caractérisés par le fait qu'ils répondent à la formule générale (VI) suivante :

$$H_2N\text{-}A\text{-}S\text{-}CO\text{-}\underset{\displaystyle R_1}{\underset{|}{C}H}\text{-}(CH_2)_m\text{-}NH_2 \quad , 2HX \quad \text{(VI)}$$

dans laquelle :
A, X, $R_1$ et m ont les mêmes significations que celles données à la revendication 4 pour la formule générale (II),
à l'exclusion des composés dans lesquels :

(i) A représente $-(CH_2)_2-$ , m est 0 ou 1 et $R_1$ représente un atome d'hydrogène,

(ii) A représente $-(CH_2)_2-$ , m est 0, $R_1$ représente un radical méthyle, éthyle, isopropyle, isobutyle, méthylthioéthyle ou benzyle.

**18.** Composés selon la revendication 17, caractérisé par le fait qu'ils sont choisis parmi :
le dichlorhydrate du thioglycinate d'amino-3 propyle,
le dichlorhydrate du thioglycinate d'amino-5 pentyle,
le dichlorhydrate du thioglycinate d'amino-2 éthoxy-2'éthyle,
le dichlorhydrate de l'amino-4 thiobutyrate d'amino-2 éthyle,
le dichlorhydrate de l'amino-4 thiobutyrate d'amino-3 propyle,
le dichlorhydrate de l'amino-4 thiobutyrate d'amino-5 pentyle,
le dichlorhydrate de l'amino-4 thiobutyrate d'(amino-2 éthoxy)-2' éthyle,
le dichlorhydrate de l'amino-6 thiocoproate d'amino-2 éthyle,
le dichlorhydrate du thioalaninate d'amino-3 propyle,
le dichlorhydrate du thioalaninate d'amino-5 pentyle, et
le dichlorhydrate du thioalaninate d'(amino-2 éthoxy)-2'éthyle.

**19.** Procédé de préparation des composés selon les revendications 17 et 18, caractérisé par le fait qu'il consiste à faire réagir un aminothiol de formule : HX, $H_2N$ - A - SH avec un halogénure d'acide d'amino acide

de formule :

$$X - CO - \underset{R_1}{\underset{|}{CH}} - (CH_2)_m - NH_2 \quad , HX$$

dans lesquelles :
A, X, $R_1$ et m ont les mêmes significations que celles données à la revendication 17, la réaction étant conduite à une température comprise entre environ 40 et 110°C en présence d'au moins un solvant organique inerte.

**20.** Procédé de déformation permanente des cheveux consistant, dans une première étape, à réduire les liaisons disulfures de la kératine par application d'une composition réductrice, puis dans une seconde étape, à reformer lesdites liaisons par application d'une composition oxydante, caractérisé par le fait que l'étape de réduction est réalisée à l'aide d'une composition cosmétique réductrice telle que revendiquée selon l'une quelconque des revendications 1 à 8.

**21.** Procédé selon la revendication 20, caractérisé par le fait que l'on laisse agir la composition réductrice pendant un temps compris entre 5 et 60 minutes.

## Patentansprüche

**1.** Kosmetische Zubereitung für den ersten Schritt eines Verfahrens zur dauerhaften Haarverformung, dadurch gekennzeichnet, daß sie in einem geeigneten kosmetischen Träger ais Reduktionsmittel wenigstens ein Aminomercaptoalkylamid der folgenden allgemeinen Formel enthält

$$HS-A-NH-CO-\underset{R_1}{\underset{|}{CH}}-(CH_2)_m-NH_2 \qquad (I)$$

worin A für den zweiwertigen Rest $-(CH_2)_n-$, worin n eine Zahl zwischen 2 und 5 ist, oder den zweiwertigen Rest $-(CH_2)_2-O-(CH_2)_2-$ steht; m 0 oder eine ganze Zahl zwischen 1 und 4 ist; und

(i) dann, wenn m 0 ist, $R_1$ für ein Wasserstoffatom, einen Methylrest, einen Ethylrest, einen Isopropylrest, einen Isobutylrest, einen 2-Methylbutylrest, einen Benzylrest, einen 4-Aminobutylrest, einen 3-Guanidinopropylrest, einen 2-Methylthioethylrest, einen Carboxymethylrest oder einen 2-Carboxyethylrest steht, und

(ii) dann, wenn m eine ganze Zahl zwischen 1 und 4 ist, $R_1$ für ein Wasserstoffatom oder einen Niederalkylrest mit 1 bis 5 Kohlenstoffatomen steht,

und die Salze der genannten Verbindungen der Formel (I).

**2.** Zubereitung nach Anspruch 1, dadurch gekennzeichnet, daß die Salze der Verbindungen der Formel (I) gewählt sind unter Hydrochloriden, Hydrobromiden, Citraten und Oxalaten.

**3.** Zubereitung nach Anspruch 1 oder 2, dadurch gekennzeichnet, daß die Verbindungen gewählt sind unter

- 3-Amino-N-(2-mercaptoethyl-)propionamid oder Alethein;
- 2-Amino-N-(2-mercaptoethyl-)acetamid;
- 2-Amino-N-(3-mercaptopropyl-)acetamid;
- 2-Amino-N-(5-mercaptopentyl-)acetamid;
- 2-Amino-N-[(2-mercaptoethoxy-)2'-ethyl-]acetamid;
- 2-Amino-N-(2-mercaptoethyl-)propionamid;
- 2-Amino-N-(3-mercaptopropyl-)propionamid;
- 2-Amino-N-(5-mercaptopentyl-)propionamid;
- 2-Amino-N-(2-mercapto-2'-ethoxyethyl-)propionamid;
- 3-Amino-N-(3-mercaptopropyl-)propionamid;
- 3-Amino-N-(5-mercaptopentyl-)propionamid;

- 3-Amino-N-[(2-mercaptoethoxy-)2'-ethyl-]propionamid;
- 2-Amino-N-(2-mercaptoethyl-)3-methylbutanamid;
- 2-Amino-N-(2-mercaptoethyl-)4-methylpentanamid;
- α-Amino-N-(2-mercaptoethyl-)hydrocinnamid;
- 4-Amino-[(2-mercaptoethyl-)amino-]5-oxo-5-pentansäure;
- 4-Amino-N-(2-mercaptoethyl-)butanamid;
- 4-Amino-N-(3-mercaptopropyl-)butanamid;
- 6-Amino-N-(5-mercaptopentyl-)butanamid;
- 4-Amino-[N-(2-mercaptoethoxy-)2'-ethyl-]butanamid; und
- 6-Amino-N-(2-mercaptoethyl-)hexanamid

sowie deren Hydrochloride.

4. Zubereitung nach den Ansprüchen 1 bis 3, dadurch gekennzeichnet, daß die Verbindung der Formel (I) in situ ausgehend von ihrer Thioester-Vorstufe mit der folgenden allgemeinen Formel (II) gebildet wird,

$$H_2N-A-S-CO-\underset{\substack{|\\R_1}}{CH}-(CH_2)_m-NH_2 \cdot 2HX \qquad (II)$$

worin A, $R_1$ und m dieselben Bedeutungen haben, wie sie in Anspruch 1 für die Verbindungen der allgemeinen Formel (I) angegeben sind, und X Cl oder Br ist.

5. Zubereitung nach einem der vorangehenden Ansprüche, dadurch gekennzeichnet, daß die Verbindung der Formel (I) oder (II) in einer Konzentration zwischen 2 und 30 Gewichtsprozent, vorzugsweise zwischen 5 und 25 Gewichtsprozent, bezogen auf das Gesamtgewicht der Zubereitung, zugegen ist.

6. Zubereitung nach einem der vorangehenden Ansprüche, dadurch gekennzeichnet, daß sie einen pH-Wert zwischen 4,5 und 11 aufweist, vorzugsweise zwischen 6 und 10, der mit Hilfe eines alkalischen Mittels erhalten wird, das gewählt ist aus Ammoniak, Mono-ethanolamin , Diethanolamin, Triethanolamin, einem Carbonat oder einem Bicarbonat eines Alkalimetalls oder von Ammonium, einem Alkalimetallhydroxid, oder mit Hilfe eines ansäuemden Mittels, das gewählt ist unter Chlorwasserstoffsäure, Essigsäure, Milchsäure, Oxalsäure oder Borsäure.

7. Zubereitung nach einem der vorangehenden Ansprüche, dadurch gekennzeichnet, daß sie außerdem wenigstens ein Reduktionsmittel enthält, das gewählt ist aus Thioglykolsäure, Glycerinmonothioglykolat oder Glykolmonothioglykolat, Cysteamin und seine $C_1$-$C_4$-Acylderivate, Cystein, N-Acetylcystein, N-(2-Mercaptoethyl-)gluconamid, β-Mercaptopropionsäure und ihre Derivate, Thiomilchsäure, Pantethein, Thioglycerin und einem Sulfit und einem Bisulfit eines Alkalimetalls oder Erdalkalimetalls.

8. Zubereitung nach einem der vorangehenden Ansprüche, dadurch gekennzeichnet, daß sie außerdem wenigstens ein kationisches Polymer, ein weichmachendes Mittel, ein Proteinhydrolysat, ein Wachs, ein opacifizierendes Mittel, einen Duftstoff, ein Färbemittel, ein nichtionisches oder kationisches Tensid, einen Alkohol, ein Behandlungsmittel oder zusätzlich ein Penetrationsmittel enthält.

9. Zubereitung nach einem der vorangehenden Ansprüche, dadurch gekennzeichnet, daß sie außerdem wenigstens ein Disulfid enthält, wobei die Zubereitung vom selbstneutralisierenden Typ ist.

10. Zubereitung nach Anspruch 9, dadurch gekennzeichnet, daß das Disulfid Dithioglykolsäure, Dithioglycerin, Cystamin, N,N'-Diacetylcystamin, Cystin oder Panthetin ist.

11. Zubereitung nach Anspruch 9, dadurch gekennzeichnet, daß das Disulfid der folgenden allgemeinen Formel (III) entspricht,

$$\left[-S-A-NH-CO-\underset{\displaystyle R_1}{\underset{|}{CH}}-(CH_2)_m-NH_2\right]_2 \qquad (III)$$

worin A, $R_1$ und m dieselben Bedeutungen haben, wie sie in Anspruch 1 für die Verbindungen der allgemeinen Formel (I) angegeben sind.

**12.** Zubereitung nach Anspruch 11, dadurch gekennzeichnet, daß das Disulfid der Formel (III) gewählt ist aus

- N,N'-(Dithiodiethan-2,1-diyl-)bis[3-aminopropionamid];
- N,N'-(Dithiodiethan-2,1-diyl-)bis[2-aminoacetamid];
- N,N'-(Dithiodiethan-2,1-diyl-)bis[2-aminopropionamid];
- N, N'-[Dithiobis(trimethylen-)]bis[2-aminopropionamid];
- N,N'-(Dithiodiethan-2,1-diyl-)bis[α-aminobenzol-propionamid];
- N,N'-(Dithiodiethan-2,1-diyl-)bis[2-amino-4-methylpentanamid];
- N,N'-(Dithiodiethan-2,1-diyl-)bis[4-aminobutanamid];
- N,N'-[Dithiobis(trimethylen-)]bis[3-aminopropionamid];
- [Dithiobis(ethan-2,1-diylimino-)]5,5'-bis[4-amino-5-oxopentansäure];
- N,N'-[Dithiobis(trimethylen-)]bis[2-aminoacetamid];
- N,N'-[Dithiobis(pentamethylen-)]bis[2-aminoacetamid];
- N,N'-[Dithiobis(pentamethylen-)]bis[4-aminobutanamid];
- N,N'-[Dithiobis(2-ethyloxyethyl-)]bis[2-aminoacetamid]; und
- N,N'-(Dithiodiethan-2,1-diyl-)bis[6-aminohexanamid].

**13.** Zubereitung nach einem der Ansprüche 8 bis 12, dadurch gekennzeichnet, daß das Disulfid in einem Molverhältnis, bezogen auf die Verbindung der Formel (I), von 0,5 bis 2,5, vorzugsweise von 1 bis 2, zugegen ist.

**14.** Neue Verbindungen, dadurch gekennzeichnet, daß sie der folgenden allgemeinen Formel (IV) entsprechen,

$$HS-A-NH-CO-\underset{\displaystyle R_1}{\underset{|}{CH}}-(CH_2)_m-NH_2 \qquad (IV)$$

worin A, $R_1$ und m dieselben Bedeutungen aufweisen, wie sie in Anspruch 1 für die allgemeine Formel (I) angegeben wurden, mit Ausnahme der Verbindungen, in denen

(i) A für $-(CH_2)_2-$ steht und m 0 oder 1 ist und $R_1$ für ein Wasserstoffatom, einen geradkettigen oder verzweigten Alkylrest mit 1 bis 5 Kohlenstoffatomen, einen Benzylrest oder einen 2-Carboxyethylrest steht, und

(ii) A für $-(CH_2)_3-$ steht und m 1 ist und $R_1$ für ein Wasserstoffatom steht, und die Salze der genannten Verbindungen der Formel (IV) sowie die entsprechenden Disulfide.

**15.** Verbindungen nach Anspruch 14, dadurch gekennzeichnet, daß die Verbindungen der Formel (IV) gewählt sind aus

- 2-Amino-N-(3-mercaptopropyl-)acetamid;
- 2-Amino-N-(5-mercaptopentyl-)acetamid;
- 2-Amino-N-[(2-mercaptoethoxy-)2'-ethyl-]acetamid;
- 2-Amino-N-(3-mercaptopropyl-)propionamid;
- 2-Amino-N-(5-mercaptopentyl-)propionamid;
- 2-Amino-N-(2-mercapto-2'-ethoxyethyl-)propionamid;
- 3-Amino-N-(3-mercaptopropyl-)propionamid;
- 3-Amino-N-(5-mercaptopentyl-)propionamid;

- 3-Amino-N-[(2-mercaptoethoxy-)2'-ethyl-]propionamid;
- 4-Amino-N-(2-mercaptoethyl-)butanamid;
- 4-Amino-N-(3-mercaptopropyl-)butanamid;
- 6-Amino-N-(5-mercaptopentyl-)butanamid;
- 4-Amino-N-[(2-mercaptoethoxy-)2'-ethyl-]butanamid; und
- 6-Amino-N-(2-mercaptoethyl-)hexanamid

sowie die Hydrochloride dieser Verbindungen.

**16.** Verbindungen nach Anspruch 14, dadurch gekennzeichnet, daß die Disulfide gewählt sind aus
- N,N'-[Dithiobis(trimethylen-)]bis[2-aminoacetamid];
- N,N'-[Dithiobis(pentamethylen-)]bis[2-aminoacetamid];
- N,N'-[Dithiobis(2-ethyloxyethyl-)]bis[2-aminoacetamid];
- N,N'-[Dithiodiethan-2,1-diyl-]bis[6-aminohexanamid];
- N,N'-[Dithiodiethan-2,1-diyl-]bis[4-aminobutanamid];
- N,N'-[Dithiobis(trimethylen-)]bis[4-aminobutanamid];
- N,N'-[Dithiobis(pentamethylen-)]bis[4-aminobutanamid];
- N,N'-[Dithiobis(trimethylen-)]bis[2-aminopropionamid];
- N,N'-[Dithiobis(trimethylen-)]bis[3-aminopropionamid];
- N,N'-[Dithiobis(pentamethylen-)]bis[3-aminopropionamid]; und
- N,N'-[Dithiobis(2-ethyloxyethyl-)]bis[3-aminopropionamid].

**17.** Neue Verbindungen, dadurch gekennzeichnet, daß sie der folgenden allgemeinen Formel (VI) entsprechen

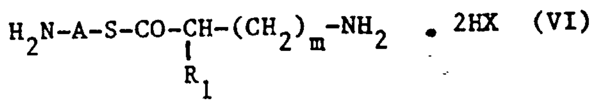

(VI)

worin A, X, $R_1$ und m die gleichen Bedeutungen haben, wie sie in Anspruch 4 für die Verbindung der allgemeinen Formel (II) angegeben sind, mit Ausnahme der Verbindungen, in denen

(i) A für $-(CH_2)_2-$ steht, m 0 oder 1 ist und $R_1$ fur ein Wasserstoffatom steht;

(ii) A für $-(CH_2)_2-$ steht, m 0 ist und $R_1$ für einen Methylrest, Ethylrest, Isopropylrest, Isobutylrest, Methylthioethylrest oder Benzylrest steht.

**18.** Verbindungen nach Anspruch 17, dadurch gekennzeichnet, daß sie gewählt sind aus
- dem Bishydrochlorid von 3-Aminopropylthioglycinat;
- dem Bishydrochlorid von 5-Aminopentylthioglycinat;
- dem Bishydrochlorid von 2-Amino-2'-ethoxyethylthioglycinat;
- dem Bishydrochlorid von 2-Aminoethyl-4-aminothiobutyrat;
- dem Bishydrochlorid von 3-Aminopropyl-4-aminothiobutyrat;
- dem Bishydrochlorid von 5-Aminopentyl-4-aminothiobutyrat;
- dem Bishydrochlorid von (2-Aminoethoxy-)2'-ethyl-4-aminothiobutyrat;
- dem Bishydrochlorid von 2-Aminoethyl-6-aminothiocaproat;
- dem Bishydrochlorid von 3-Aminopropylthioalaninat;
- dem Bishydrochlorid von 5-Aminopentylthioalaninat; und
- dem Bishydrochlorid von (2-Aminoethoxy-)2'-ethylthioalaninat.

**19.** Verfahren zur Herstellung der Verbindungen nach den Ansprüchen 17 und 18, dadurch gekennzeichnet, daß es daraus besteht, ein Aminothiol der Formel

$$HX . H_2N - A - SH$$

mit einem Hydrohalogenid einer Aminosäure der Formel

$$X - CO - \underset{\displaystyle R_1}{CH} - (CH_2)_m - NH_2 \cdot HX$$

umzusetzen, worin A, X, $R_1$ und m die gleichen Bedeutungen haben, wie sie in Anspruch 17 angegeben sind, wobei die Reaktion bei einer Temperatur zwischen etwa 40 und 110°C in Gegenwart wenigstens eines inerten organischen Lösungsmittels durchgeführt wird.

**20.** Verfahren zur dauerhaften Verformung der Haare, das in einem ersten Schritt daraus besteht, die Disulfid-Bindungen des Keratins durch Aufbringung einer reduzierenden Zubereitung zu reduzieren und danach in einem zweiten Schritt die Bindungen erneut durch Aufbringung einer oxidierenden Zubereitung zu bilden, dadurch gekennzeichnet, daß der Reduktionsschritt mit Hilfe einer kosmetischen reduzierenden Zubereitung durchgeführt wird, wie sie in einem der Ansprüche 1 bis 8 beansprucht ist.

**21.** Verfahren nach Anspruch 20, dadurch gekennzeichnet, daß man die reduzierende Zubereitung während einer Zeit zwischen 5 und 60 Minuten einwirken läßt.

## Claims

**1.** Cosmetic composition for the first stage of an operation of permanent-reshaping of hair, characterised in that it contains, in a suitable cosmetic vehicle, as a reducing agent, at least one amino(mercaptoalkyl)amide having the following general formula:

$$HS-A-NH-CO-\underset{\displaystyle R_1}{CH}-(CH_2)_m-NH_2 \qquad (I)$$

in which:

A represents the divalent radical $-(CH_2)_n-$, n being an integer between 2 and 5, or the divalent radical $-(CH_2)_2-O-(CH_2)_2-$

m is 0 or an integer between 1 and 4,

(i) when m is 0, $R_1$ represents a hydrogen atom, a methyl radical, an ethyl radical, an isopropyl radical, an isobutyl radical, a 2-methylbutyl radical, a benzyl radical, a 4-aminobutyl radical, a 3-guanidinopropyl radical, a 2-(méthylthio)ethyl radical, a carboxymethyl radical or a 2-carboxyethyl radical,

(ii) when m is an integer from 1 to 4, $R_1$ represents a hydrogen atom or a lower alkyl radical having from 1 to 5 carbon atoms,

and the salts of the said compounds of formula (I).

**2.** Composition according to Claim 1, characterised in that the salts of the compounds of formula (I) are chosen from the hydrochlorides, hydrobromides, citrates and oxalates.

**3.** Composition according to Claim 1 or 2, characterised in that the compounds are chosen from:

- 3-amino-N-(2-mercaptoethyl)propionamide or aletheine,
- 2-amino-N-(2-mercaptoethyl)acetamide,
- 2-amino-N-(3-mercaptopropyl)acetamide,
- 2-amino-N-(5-mercaptopentyl)acetamide,
- 2-amino-N-[2'-(2-mercaptoethoxy)ethyl]acetamide,
- 2-amino-N-(2-mercaptoethyl)propionamide,
- 2-amino-N-(3-mercaptopropyl)propionamide,
- 2-amino-N-(5-mercaptopentyl)propionamide,
- 2-amino-N-[2'-(2-mercaptoethoxy)ethyl]propionamide,
- 3-amino-N-(3-mercaptopropyl)propionamide,
- 3-amino-N-(5-mercaptopentyl)propionamide,

- 3-amino-N-[2'-(2-mercaptoethoxy)ethyl-]propionamide,
- 2-amino-N-(2-mercaptoethyl)-3-methylbutanamide,
- 2-amino-N-(2-mercaptoethyl)-4-methylpentanamide,
- α-amino-N-(2-mercaptoethyl)hydrocinnamide,
- 4-amino-5-[(2-mercaptoethyl)amino]-5-oxopentanoic acid,
- 4-amino-N-(2-mercaptoethyl)butanamide,
- 4-amino-N-(3-mercaptopropyl)butanamide,
- 6-amino-N-(5-mercaptopentyl)butanamide,
- 4-amino-N-[2'-(2-mercaptoethoxy)ethyl]butanamide, and
- 6-amino-N-(2-mercaptoethyl)hexanamide,

as well as their hydrochlorides.

4. Composition according to Claims 1 to 3, characterised in that the compound of formula (I) is formed in situ from its thioester precursor having the following general formula (II):

$$H_2N-A-S-CO-\underset{\displaystyle R_1}{\underset{|}{CH}}-(CH_2)_m-NH_2 \ . \ 2HX \qquad (II)$$

in which:

A, $R_1$ and m have the same meanings as those given in Claim 1 for the general formula (I), and X is Cl or Br.

5. Composition according to any one of the preceding claims, characterised in that the compound of formula (I) or (II) is present at a concentration of between 2 and 30 %, and preferably between 5 and 25 %, by weight relative to the total weight of the composition.

6. Composition according to any one of the preceding claims, characterised in that it possesses a pH of between 4.5 and 11, and preferably between 6 and 10, obtained using an alkaline agent chosen from ammonia solution, monoethanolamine, diethanolamine, triethanolamine, an alkali metal or ammonium carbonate or bicarbonate or an alkali metal hydroxide, or using an acidifying agent chosen from hydrochloric acid, acetic acid, lactic acid, oxalic acid or boric acid.

7. Composition according to any one of the preceding claims, characterised in that it contains, in addition, at least one other reducing agent chosen from: thioglycolic acid, glyceryl monothioglycolate or glycol monothioglycolate, cysteamine and its $C_1$-$C_4$ acyl derivatives, cysteine, N-acetylcysteine, N-(2-mercaptoethyl)-gluconamide, β-mercaptopropionic acid and its derivatives, thiolactic acid, pantetheine, thioglycerol and an alkali metal or alkaline earth metal sulphite and bisulphite.

8. Composition according to any one of the preceding claims, characterised in that it contains, in addition, at least one cationic polymer, one emollient agent, one protein hydrolysate, one wax, one opacifying agent, one perfume, one colorant, one nonionic or cationic surfactant, one alcohol, one treating agent or alternatively one penetrating agent.

9. Composition according to any one of the preceding claims, characterised in that it contains, in addition, at least one disulphide, the composition being of the self-neutralising type.

10. Composition according to Claim 9, characterised in that the disulphide is dithioglycolic acid, dithioglycerol, cystamine, N,N'-diacetylcystamine, cystine or pantethine.

11. Composition according to Claim 9, characterised in that the disulphide corresponds to the following general formula (III):

$$\left[\text{—S-A-NH-CO-}\underset{\displaystyle R_1}{\underset{|}{\text{CH}}}\text{-(CH}_2\text{)}_m\text{-NH}_2\right]_2 \qquad \text{(III)}$$

in which:
A, $R_1$ and m have the same meanings as those given in Claim 1 for the general formula (I).

**12.** Composition according to Claim 11, characterised in that the disulphide of formula (III) is chosen from:
N,N'-[dithiobis(ethylene)]bis[3-aminopropionamide],
N,N'-[dithiobis(ethylene)]bis[2-aminoacetamide],
N,N'-[dithiobis(ethylene)]bis[2-aminopropionamide],
N,N'-[dithiobis(trimethylene)]bis[2-aminopropionamide],
N,N'-[dithiobis(ethylene)]bis[α-aminobenzenepropionamide],
N,N'-[dithiobis(ethylene)]bis[2-amino-4-methylpentanamide],
N,N'-[dithiobis(ethylene)]bis[4-aminobutanamide],
N,N'-[dithiobis(trimethylene)]bis[3-aminopropionamide],
5,5'-[dithiobis(ethylenimino)]bis[4-amino-5-oxopentanoic acid],
N,N'-[dithiobis(trimethylene)]bis[2-aminoacetamide],
N,N'-[dithiobis(pentamethylene)]bis[2-aminoacetamide],
N,N'-[dithiobis(pentamethylene)]bis[4-aminobutanamide],
N,N'-[dithiobis(2-ethyloxyethyl)]bis[2-aminoacetamide], and
N,N'-[dithiobis(ethylene)]bis[6-aminohexanamide].

**13.** Composition according to any one of Claims 8 to 12, characterised in that the disulphide is present in a molar proportion relative to the compound of formula (I) ranging from 0.5 to 2.5, and preferably from 1 to 2.

**14.** New compounds, characterised in that they correspond to the following general formula (IV):

$$\text{HS-A-NH-CO-}\underset{\displaystyle R_1}{\underset{|}{\text{CH}}}\text{-(CH}_2\text{)}_m\text{-NH}_2 \qquad \text{(IV)}$$

in which:
A, $R_1$ and m have the same meanings as those given in Claim 1 for the general formula (I), with the exception of the compounds in which:
(i) A represents $-(CH_2)_2-$ and m is 0 or 1, and $R_1$ represents a hydrogen atom, a linear or branched alkyl radical having from 1 to 5 carbon atoms, a benzyl radical or a 2-carboxyethyl radical, and
(ii) A represents $-(CH_2)_3-$ and m is 1, and $R_1$ represents a hydrogen atom, and the salts of the said compounds of formula (IV) as well as their corresponding disulphides.

**15.** Compounds according to Claim 14, characterised in that the compounds of formula (IV) are chosen from:
2-amino-N-(3-mercaptopropyl)acetamide,
2-amino-N-(5-mercaptopentyl)acetamide,
2-amino-N-[2'-(2-mercaptoethoxy)ethyl]acetamide,
2-amino-N-(3-mercaptopropyl)propionamide,
2-amino-N-(5-mercaptopentyl)propionamide,
2-amino-N-[2'-(2-mercaptoethoxy)ethyl]propionamide,
3-amino-N-(3-mercaptopropyl)propionamide,
3-amino-N-(5-mercaptopentyl)propionamide,
3-amino-N-[2'-(2-mercaptoethoxy)ethyl]propionamide,
4-amino-N-(2-mercaptoethyl)butanamide,

4-amino-N-(3-mercaptopropyl)butanamide,
6-amino-N-(5-mercaptopentyl)butanamide,
4-amino-N-[2'-(2-mercaptoethoxy)ethyl]butanamide, and
6-amino-N-(2-mercaptoethyl)hexanamide,
as well as their hydrochlorides.

**16.** Compounds according to Claim 14, characterised in that the disulphides are chosen from:
N,N'-[dithiobis(trimethylene)]bis[2-aminoacetamide],
N,N'-[dithiobis(pentamethylene)]bis[2-aminoacetamide],
N,N'-[dithiobis(2-ethyloxyethyl)]bis[2-aminoacetamide],
N,N'-[dithiobis(ethylene)]bis[6-aminohexanamide],
N,N'-[dithiobis(ethylene)]bis[4-aminobutanamide],
N,N'-[dithiobis(trimethylene)]bis[4-aminobutanamide],
N,N'-[dithiobis(pentamethylene)]bis[4-aminobutanamide],
N,N'-[dithiobis(trimethylene)]bis[2-aminopropionamide],
N,N'-[dithiobis(trimethylene)]bis[3-aminopropionamide],
N,N'-[dithiobis(pentamethylene)]bis[3-aminopropionamide], and
N,N'-[dithiobis(2-ethyloxyethyl)]bis[3-aminopropionamide].

**17.** New compounds, characterised in that they correspond to the following general formula (VI):

$$H_2N-A-S-CO-\underset{\displaystyle R_1}{\underset{|}{CH}}-(CH_2)_m-NH_2 \cdot 2HX \qquad (VI)$$

in which:
A, X, $R_1$ and m have the same meanings as those given in Claim 4 for the general formula (II), with the exception of the compounds in which:
(i) A represente $-(CH_2)_2-$, m is 0 or 1 and $R_1$ represents a hydrogen atom,
(ii) A repreeents $-(CH_2)_2-$, m is 0 and $R_1$ represents a methyl, ethyl, isopropyl, isobutyl, methylthioethyl or benzyl radical.

**18.** Compounds according to Claim 17, characterised in that they are chosen from:
3-aminopropyl thioglycinate dihydrochloride,
5-aminopentyl thioglycinate dihydrochloride,
2'-(2-aminoethoxy)ethyl thioglycinate dihydrochloride,
2-aminoethyl 4-aminothiobutyrate dihydrochloride,
3-aminopropyl 4-aminothiobutyrate dihydrochloride,
5-aminopentyl 4-aminothiobutyrate dihydrochloride,
2'-(2-aminoethoxy)ethyl 4-aminothiobutyrate dihydrochloride,
2-aminoethyl 6-aminothiocaproate dihydrochloride,
3-aminopropyl thioalaninate dihydrochloride,
5-aminopentyl thioalaninate dihydrochloride, and
2'-(2-aminoethoxy)ethyl thioalaninate dihydrochloride.

**19.** Process for preparing the compounds according to Claims 17 and 18, characterised in that it consists in reacting an aminothiol of formula: $HX . H_2N - A - SH$ with an acid halide of an amino acid of formula:

$$X - CO - \underset{\displaystyle R_1}{\underset{|}{CH}} - (CH_2)_m - NH_2 \cdot HX$$

in which formulae:
A, X, $R_1$ and m have the same meanings as those given in Claim 17, the reaction being performed at a temperature of between approximately 40 and 110°C in the presence of at least one inert organic solvent.

**20.** Process for the permanent-reshaping of hair, consisting, in a first step, in reducing the disulphide bonds of the keratin by application of a reducing composition, and then, in a second step, in re-forming the said bonds by application of an oxidising composition, characterised in that the reduction step is carried out using a reducing cosmetic composition as claimed in any one of Claims 1 to 8.

**21.** Process according to Claim 20, characterised in that the reducing composition is left to act for a time of between 5 and 60 minutes.